# EUROPEAN PATENT SPECIFICATION

(11) **EP 2 863 798 B1**
(45) Date of publication and mention of the grant of the patent: **02.05.2018**
(21) Application number: 13734865.2
(22) Date of filing: 20.06.2013
(51) Int. Cl.: G01N 33/49, G01N 1/40, G01N 33/543, A61B 5/15, B01L 3/00

(54) **QUICK TEST DEVICE AND METHOD**
SCHNELLTESTVORRICHTUNG UND -VERFAHREN
DISPOSITIF DE TEST RAPIDE ET PROCÉDÉ

(30) Priority: 20.06.2012 EP 12172828
(43) Date of publication of application: 29.04.2015
(73) Proprietor: Fabpulous B.V., 6229 EV Maastricht (NL)
(72) Inventor: ENGBERSEN, Diederik Justus Marie, NL-6229 EV Maastricht (NL); MOHREN, Ronny Johannes Cunegonda, NL-6229 EV Maastricht (NL); ZEIS, John Kenneth, NL-6229 EV Maastricht (NL)
(74) Representative: V.O.
(86) International application number: PCT/NL2013/050442
(87) International publication number: WO 2013/191552

(56) References cited:
- WO-A1-2009/064079
- WO-A2-2007/091987
- DE-A1-102009 007 616
- US-A- 4 635 488
- US-A1- 2004 014 203
- US-A1- 2005 180 882

## Description

The invention relates to a test device for testing blood. The invention further relates to a method for testing blood. The invention especially is related to such device and method for quick testing of blood.

In EP 2 283 360 a quick test device for blood is disclosed, wherein a sponge is saturated with blood absorbed from a prick made in a person's finger. The sponge is inserted into a cylindrical holder, in which an amount of dilutant is provided, such that the blood is rinsed from the sponge and diluted. Then a plunger is inserted into the cylinder, having a filter at the free end thereof. When the plunger is pressed into the cylinder, the diluted blood is forced at least partly through the filter into a space within the plunger. The filter is designed to filter blood cells from the diluted blood, such that blood plasma passes the filter and the red blood cells are retained. Within the plunger a reagent is provided, for contacting the blood plasma. The cylinder and the plunger are transparent, such that the reaction, if any, with the reagent can be seen through the cylinder. An inner plunger is then forced down into the plunger, such that a stop is forced into the filter opening, closing said opening.

This known device has the disadvantage that it is complicated in construction and use, is costly and the forcing of the blood through the filter is difficult to control. The device allows the blood to be pressed through the filter at too high a pressure, which can lead to high shear on the blood cells, contaminating the serum obtained after filtering. A further disadvantage of this known device is that the results can be inconsistent, which appears to be the result of at least the inter-user variability and inconsistency of the filtrate of the whole blood sample.

US2004/014203 discloses a test device of a sample such as blood, comprising a first part that can be coupled to a second part. The first part comprises a reservoir containing a buffer fluid and a space for receiving a sample. The reservoir can be pushed into the said space, opening the reservoir and allowing the buffer container therein to flow into the space. The second part comprises a test strip and a filter, with which filter the test strip is held in place. A liquid sample can be entered into the space through an open end thereof, to be closed off by the reservoir, or can be sucked into the space by a suction element. The buffer fluid will be mixed with the sample inside the space and will then flow under gravity and capillary action onto the strip, towards a reagent, The flow is controlled by the filter.

An aim of the disclosure is to provide an alternative device for testing human or animal samples such as blood and/or components thereof, especially a quick testing device. An aim of the present disclosure is to provide compressed, a weakened portion of the buffer container will fail and buffer will contact the sample and then flow through a lumen to and through the filter to the test strip. The filter is in direct contact with the test strip, such that the buffer with sample is drawn through the filter. In this known device the flow of the diluted sample through the filter is by passive, capillary flow.

An aim of the present disclosure is to provide an alternative device for testing human or animal samples such as blood and/or components thereof, especially a quick testing device. An aim of the present disclosure is to provide an easy to use device. An aim of the present disclosure is to provide for a test device allowing proper control of the pressure build up therein and of the filtering of a sample, such as blood, resulting in a consistent output and test result.

In an aspect a test device of this disclosure is characterised by the features of claim 1. The adsorbing and/or absorbing element is comprised in the first part in a position to receive the sample, for example blood from for example a prick in a finger.

A device of the invention allows the fluid to be forced into and/or through the element, to dissolve and/or dilute the blood prior to being forced through the filter. This can reduce the risk of damaging the sample, especially blood cells, especially the red blood cells. The reservoir, especially the construction for reducing the volume of the reservoir and thus defining the amount of fluid to be passed through the element, and the element define specific amounts of the sample, such as blood and fluid passed through the filter, such that the dilution factor is defined. This can increase the accuracy of the reaction with the reagent. According to the invention, the first part comprises a mechanism for opening the reservoir and controlled reduction of the volume of the reservoir for forcing liquid from the reservoir. The control mechanism will prevent pressure exerted on the fluid from being too high even better. The mechanism can for example comprise co-operating elements transferring a rotation of a grip into a translation of a part of the device for reducing the volume of the reservoir. Surprisingly such conversion mechanism can prevent a too fast reduction of the volume of the reservoir. In a preferred embodiment, the first part comprises a seal sealing off the reservoir, wherein the first part is provided with an element for piercing or rupturing the seal, such that a fluid connection is formed between the reservoir and the adsorbing and/or absorbing element.

The seal closes off the reservoir, such that the fluid contained therein cannot leave the reservoir before the seal is pierced or ruptured. This will prevent fluid from leaving the first part too early, for example when the element is not in place or when the first part is not properly connected to the second part. Moreover this allows easy storage of the device, especially the first part, prior to use.

In a further aspect the present disclosure is characterised by a method for testing according to claim 15 or preparing for testing according to claim 17 a human or animal sample, such as blood. The part of the diluted and/or dissolved sample passing through the filter as a filtrate can then at least partly be brought into contact with the at least one reagent, for reaction therewith. This can be done directly, in a part connected to the channel passed the filter or can be done separate thereof. To such end the filtrate can for example be received in a receptacle such as a container to be dispatched to a location for testing, can be stored for later reference or otherwise treated separate from the part comprising the channel.

In a preferred embodiment the at least one reagent is provided in or on a strip of material in a second part of the device, wherein the strip is connected to the filter or at least to a chamber in or in connection with which the filter is provided. The strip can be made of any suitable material and can for example be made of an absorbent material, a material providing transport of the diluted fluid combination to the at least one reagent. The material may define at least partly the speed of transportation of the fluid along the strip, for defining a reaction time of the fluid with the reagent. The material can for example further separate constituents from the fluid combination, in order to ensure the correct reaction with the reagent even better.

In preferred embodiment, the test device is provided with a sample taking element, especially a blood absorbing and/or adsorbing blood, wherein a quantity of the sample such as blood is adsorbed and/or absorbed in and/or on an element, which at one side is connected to a reservoir containing a liquid, especially a solvent or dilutant for the sample, such as a dilutant for blood. The element is then connected to either a receptacle for storage and/or transport of the diluted sample or filtrate or to a channel comprising at least one reagent and/or a filter. The reservoir is reduced in volume, such that the liquid contained therein is at least partly forced through and/or along the element, dissolving and/or diluting the sample therein and/or thereon. The then diluted and/or dissolved sample is forced at least partly through the filter, wherein in the filter at least specific cells such as specific blood cells, especially red blood cells are filtered out. The part of the diluted and/or dissolved sample passing through the filter as a filtrate can then at least partly be brought into contact with the at least one reagent, for reaction therewith. This can be done directly, in a part connected to the channel passed the filter or can be done separate thereof. To such end the filtrate can for example be received in a receptacle such as a container to be dispatched to a location for testing, can be stored for later reference or otherwise treated separate from the part comprising the channel.

In a preferred embodiment the at least one reagent is provided in or on a strip of material in a second part of the device, wherein the strip is connected to the filter or at least to a chamber in or in connection with which the filter is provided. The strip can be made of any suitable material and can for example be made of an absorbent material, a material providing transport of the diluted fluid combination to the at least one reagent. The material may define at least partly the speed of transportation of the fluid along the strip, for defining a reaction time of the fluid with the reagent. The material can for example further separate constituents from the fluid combination, in order to ensure the correct reaction with the reagent even better. In a preferred embodiment, a test device is provided with a sample taking element, especially a blood absorbing and/or adsorbing element, for taking a blood sample, and preferably a filter for filtering out elements from the sample, wherein at least one of the blood absorbing and/or adsorbing element and the filter is provided with a reactive component reactive with components of the sample and/or components on, in or of a test strip. Such reactive component can for example be a reagent, an agent, a conjugate or a blocking agent. In accordance with the present invention. there is also provided a kit of parts for a test device as defined in claim 18.

Further advantages are achieved by the embodiments indicated by the dependent claims.

In further elucidation of the present disclosure embodiments of a device and method for quick testing shall be described hereafter, with reference to the drawings. Therein shows:
Fig. 1 in perspective view an embodiment of a test device, with a first and second part separated;
Fig. 2 a cross sectional view part of the device of fig. 1, with the first and second part in coupled condition and with the reservoir in a first position;
Fig. 3 a cross sectional view part of the device, comparable to fig. 2, with the first and second part in coupled condition and with the reservoir in a second, reduced volume position;
Fig. 4 and 5 in perspective side view part of the device in the positions of fig. 2 and 3 respectively, partly transparent;
Fig. 6 in perspective view a first part of a device during a step of adsorbing and/or absorbing blood from a finger;
Fig. 7 in cross sectional view an alternative embodiment of a device of the disclosure, in a first position;
Fig. 8 in perspective side view and cross sectional view an alternative embodiment, with the grip and reservoir in a first position;
Fig. 9 in perspective side view and cross sectional view the embodiment of fig. 8, with the grip and reservoir in an intermediate position;
Fig. 10 in perspective side view and cross sectional view the embodiment of fig. 8 and 9, with the grip and reservoir in a second, reduced volume position.
Fig. 11a, b in cross sectional view an other embodiment of the device of fig. 1, with the first and second part in coupled condition and with the reservoir in a first position and a cross sectional view part of said device with the first and second part in coupled condition and with the reservoir in a second, reduced volume position
Fig. 12 a cross sectional view with the first part of the device according to figure 11, and a receptacle.

In this description embodiments of the present invention are disclosed by way of example only. They should by no means be understood as limiting the scope of the invention as defined by the appended claims. In this description the same or similar elements or features shall be indicated by the same or similar reference signs. In this description different parts or elements are described as parts of a device or for use in a method for testing. However, these parts and elements are also considered to have been fully disclosed individually, whereas also different combinations of these parts or elements are considered to have been disclosed herein.

In this description devices and methods are described with reference to testing of blood, wherein blood is filtered in order to separate out from the blood at least most and preferably substantially all of the red blood cells. Filters can be chosen such that other elements are or can be separated out from the blood, such as blood platelets or white blood cells, either together with or in stead of the red blood cells. Moreover other human or animal samples can be used in devices and/or methods disclosed herein. Blood is only discussed by way of example.

In this description devices and methods are described wherein a sample such as blood is diluted with a fluid, prior to being forced through a filter for filtering out at least one kind of cells, especially red blood cells. Preferably the sample, especially blood is washed from and/or out of an element in and/or on which it is adsorbed and/or absorbed, such as for example but not limited to a sponge like element. Preferably the fluid is pressurised before or for being forced into and/or through said element for diluting the blood. The fluid can be a diluent, also referred to as dilutant, or a solvent for a sample taken to be tested. The element is preferably an element that can receive a predetermined amount of a sample, such as blood, especially whole blood, such as a sponge like element, a capillary element or the like, through which fluid can be passed. Adsorbing and/or absorbing has to be understood as including but not limited to receiving a sample such as blood by creating a pressure difference over the element and/or by capillary effects in and/or through said element and/or by reversible chemical and/or physical bonding of the sample, such as blood to the element.

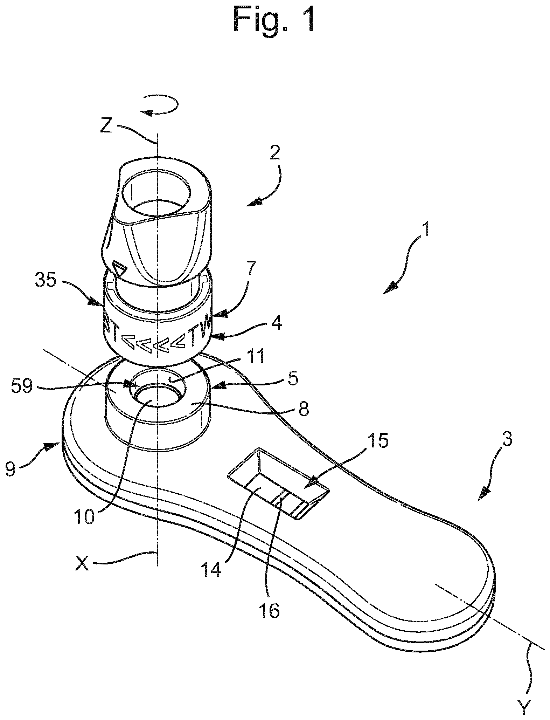

Fig. 1 shows a device 1 for performing a quick blood test. The device 1 comprises a first part 2 and a second part 3, which can be coupled to each other by a first coupling element 4 on the first part 2 and a second coupling part 5 provided on the second part 3. In the embodiment shown in fig. 1 the first coupling element is or comprises a cylindrical element 6 at a first end 7 of the first part 2, the cylindrical part having a longitudinal axis Z. The second coupling element 5 in this embodiment is formed by or comprises a substantially cylindrical wall 8 formed at or near one longitudinal end 9 of the second part 3. The wall 8 encloses a substantially cylindrical opening 59 with a first longitudinal axis X. The second part 3 is or comprises a receptacle for receiving at least part of a diluted sample such as a filtrate from the first part as will be described hereafter.

In the embodiment shown in fig. 1 - 6 and 8 - 10 the second part 3 is substantially plate shaped and has a second longitudinal axis Y, extending substantially perpendicular to the first longitudinal axis X. The opening has a bottom 10 opposite an open side 11. In the bottom 10 an outlet opening is provided in which a filter 12 is placed. At a side of the filter 12 opposite the bottom 10 a channel 13 connects to the filter 12, which extends in the second longitudinal direction Y. In the channel a strip 14 of materials is provided, for example as generally known from lateral flow essays, which can for example be absorbent materials as used in chromatographic applications, in known test strips or the like. By way of example the materials can for example be glass and/or cellulose fiber for example provided by Millipore US and/or nitrocellulose, for example with a thickness between 140-170um and a porosity of between 90-135 sec/4cm provided by Sartorius. In the second part 3 a window 15 can be provided, opening to the channel 13 and the strip 14. The window 15 is spaced apart from the second coupling element 5. At the position of the window 15 the at least one reagent 16 is provided in and/or on the strip 14, such that any reaction of the components of blood brought into contact with the at least one reagent 16 can be seen through said at least one window 15.

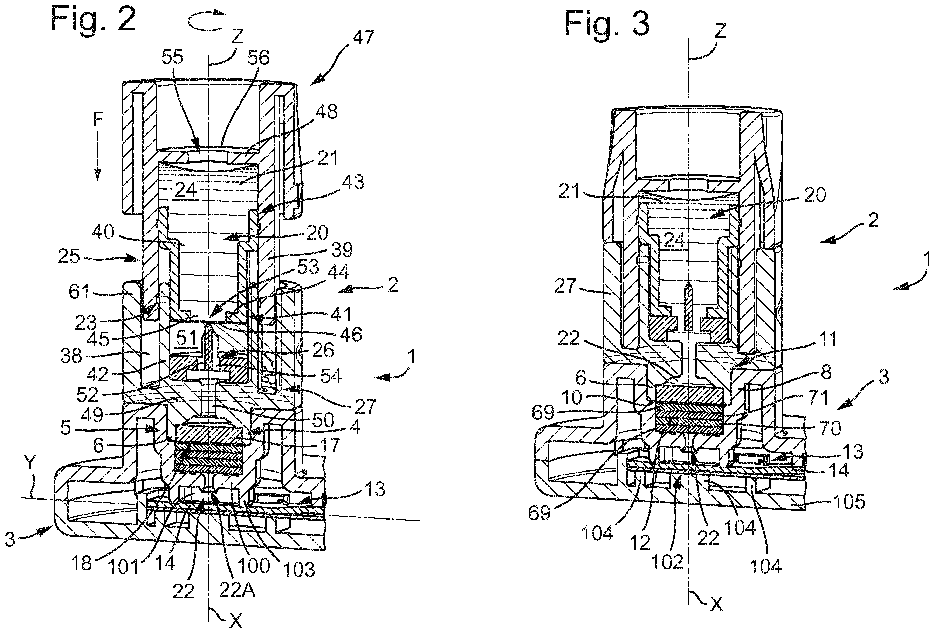

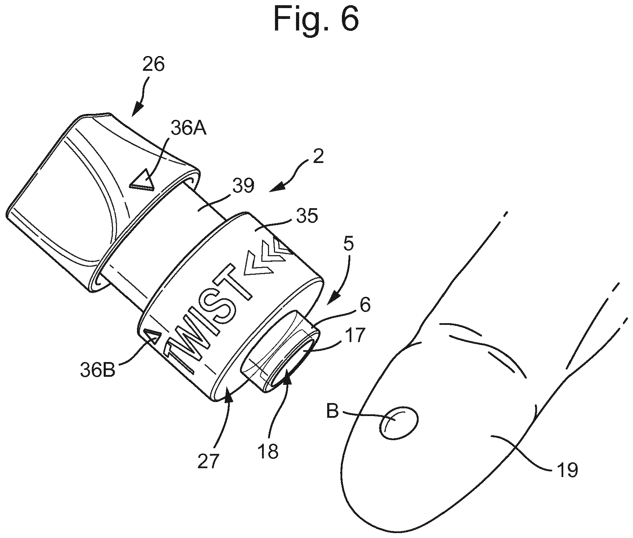

In the first part 2 a blood absorbing and/or adsorbing element 17 is provided. This element 17 can for example be formed by or comprise a sponge like element 17 such as but not limited to an artificial element having open cells, pores or the like for absorbing an amount of blood, especially full blood. Preferably the type and volume of the material of the element 17 is chosen such that the element 17 can absorb a predefined amount of full blood. The element 17 in this embodiment is positioned at the first end 7 of the first part 2, within the cylindrical part 6. A surface 18 of the element 17 can form an end portion of the first part 2 and can lie free at said end 7, such that, as is seen in fig. 6, blood B can be adsorbed directly by the element 17 from for example a prick in a finger 19. Alternatively blood can be absorbed from a different source, such as a vial. When the first part 2 is inserted into the second part 3, the first and second coupling elements 4, 5 coupled, as is for example shown in fig. 2 and 3, the element 17 is positioned directly over and preferably in close proximity or even in contact with the filter 12. The first element 4 can for example be press fit into the second element 5 or vice versa.

In this embodiment the element 17 is fixed in the first part 2. Alternatively the element 17 can be inserted into the first part 2 after having been saturated with blood.

The first part 2 comprises a fluid reservoir 20 at a side of the element 17 facing away from the free surface 18 thereof, such that any fluid coming from said reservoir 20 can only leave the first part through the element 17 and through the filter 12 when the first and second part 2, 3 are connected properly. The reservoir 20 is reducible in volume, in order to force an amount of fluid 21 contained therein out, through the element 17 and the filter 12. This has the advantage that al fluid leaving the reservoir 20 is forced through the element 17 and thus dilutes the blood absorbed therein before passing through the filter 12. The reservoir 20 is preferably provided opposite the free surface 18 thereof. Preferably the first part 2 is provided with a mechanism 23 for guiding the reduction of the volume between a maximum volume and a minimum volume or maximally reduced volume. Preferably the volume of fluid 21 in the reservoir 20 is chosen such that in the maximum volume position the reservoir is substantially entirely filled with fluid. By defining a maximally reduced volume, the amount of fluid being forced through the element 17 is thus defined and therefore the dilution rate is defined. This may increase the accuracy of the test.

When the reservoir in the maximally reduced volume position a fluid connection 22 is provided between the reservoir 20 and the at least one reagent 16 through the adsorbing and/or absorbing element 17 and the filter 12 provided between the element 17 and the at least one reagent 16. The fluid connection 22 is substantially entirely filled with the fluid 21 from the reservoir 20 and blood components contained therein. In the embodiment in which the filter 12 is designed for filtering out at least red blood cells, the fluid in the channel 13 will substantially be diluted blood plasma. In the embodiment in which the mechanism 23 is designed for controlling the maximum and minimum volume and therefore to define the amount of fluid 21 forced out of the reservoir 20, the dilution ratio is well defined and can for example be between 1:1 and 1:10 (volume of blood versus volume of fluid passed through the filter), for example a dilution ratio between 1:2 and 1: 8, such as but not limited to 1:5, meaning that four parts of fluid are added to one part of full blood. The dilution ratio can also be defined as volume of plasma obtained as or in the filtrate versus volume of fluid passed through the filter, which can for example be, but is not limited to, between 1:1 and 1:20.

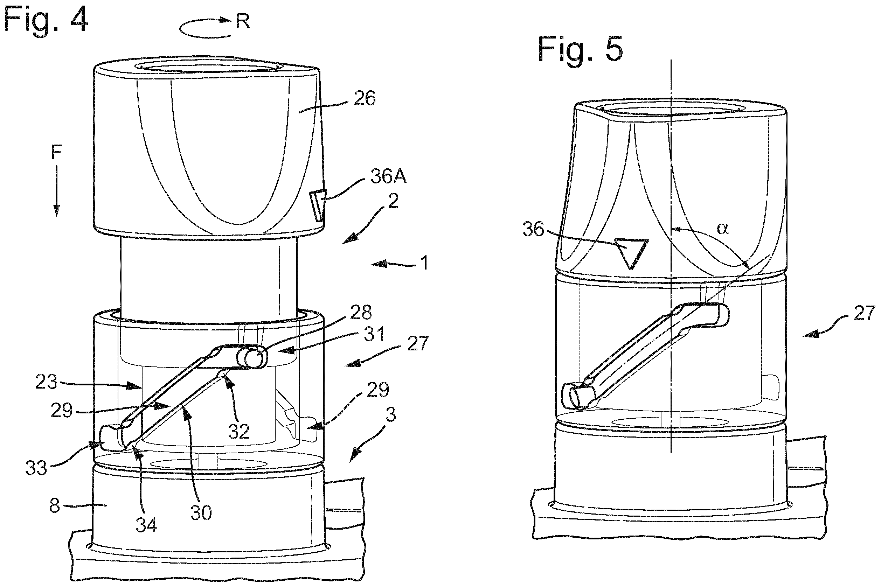

In the embodiment shown the fluid reservoir 20 can comprise a chamber 24 and a plunger 25, movable relative to said filter 12 for reducing the volume of the reservoir 20. An outlet 26 is provided for the reservoir 20, opening toward the at least one adsorbing and/or absorbing element 17, preferably at a side thereof facing away from the surface 18 and the second part 3. The plunger 25 can move in a direction F parallel to the longitudinal direction X, from a first, start position as shown in fig. 2 and 4 to a second, end position as shown in fig. 3 and 5. The reservoir 20 is basically defined between the plunger 25 and the walls of the chamber 24, and has a maximum volume when the plunger 25 is in the start position and has a minimum volume when the plunger 25 is in the end position.

The movement of the plunger 25 in the direction F can be a translation in said direction F only. Preferably the mechanism 23 will be designed such that the possible speed of the plunger 25 is limited in order to reduce the risk to overpressure the fluid. To this end for example the plunger can be biased into an opposite direction by for example a spring working on the plunger, such that a relatively large force is necessary for moving the plunger. In an alternative embodiment the plunger may be provided with a channel for passing the fluid 21 from the reservoir 20 to the element 17, such that increasing speed will lead to an increased counter pressure on the plunger 25, thus limiting the pressure of the fluid entering into the element 17. In a further embodiment the element 17 may be designed such that an increasing fluid pressure will compress the element to such extend that the debit of fluid passing there through may be reduced, such that excess pressure of fluid on the filter may again be prevented. The pressure on the filter should preferably be such that the red blood cells are not damaged. Lysis, especially but not limited to lysis of the red blood cells may interfere with an accurate test result and should therefore be avoided as much as possible.

In the embodiment shown in fig. 2 - 5 the mechanism 23 is designed to initiate and/or combine the movement of the plunger 25 in the direction F with at least a rotational movement of the plunger 25 and/or of a grip 26 or vice versa. In the embodiment shown the first part is provided with a grip 26, rotatable on or around a housing part 27 of the first part 2 comprising at least the chamber 24 and/or the plunger 25, such that a rotation of said grip 26 in direction R results in reducing the volume of the reservoir, especially of chamber 24. The plunger 25 can be directly connected to the grip 26 or can be an integral part thereof, such that the rotational movement of the grip 26 will also rotate the plunger 25. In the embodiment shown, the plunger is formed by a plunger element.

In the embodiment shown in for example fig. 2 - 5 the grip 26 is provided with at least one tooth 28 extending radially outward from a wall 39, and the housing part 27 with a guide track 29 for said tooth 28. Alternatively the tooth 28 could be provided in the housing part 27 and the track 29 on the grip 26 extending around the housing part 27. The guide track 29 extends such that a rotation R of the grip around the axis Z leads to a forced movement of the grip 26 in a linear direction F. The rotation in the direction R will lead to a movement towards the adsorbing and/or absorbing element 17. To this end the track 29 has at least a mid-section 30 extending at an angle α relative to the axis Z. The track 29 can extend at least partly as part of a spiral around part of the housing part 27. In embodiments there can be two or more such tracks 29 and corresponding teeth 28. In embodiments at a first end 31 the track 29 can be provided with a restriction 32, such that the tooth 28 has to be forced through the restriction 32 in order for the tooth to enter into the mid-section 30. This means that a user, when rotating the grip 26 out of the first or start position will have a tactile feedback. The restriction 32 will moreover reduce the risk of the grip 26 being rotated unintentionally, and thus unintentional movement of the plunger 25 in the direction F can be prevented. Similarly at the opposite second end 33 of the track 29 a restriction 34 can be provided, such that the tooth 28 has to be forced passed the restriction 34 at the end of the mid-section 30 in order to be brought into the end position, again providing tactile feedback to the user for reaching the end position.

As can be seen in especially fig. 1, 4 and 5 the track or tracks 29 can extend through the housing part 27, which will provide for easy manufacturing, for example by injection moulding. A cover 35 can then be applied over the grip, covering the tracks 29. The cover 35 can for example be a foil. The grip 26 and the housing part 27 can for example be provided with visual indicators 36, indicating when the start position and/or the end position are reached. In the embodiment shown the indicators 36 are designed as arrows 36A, B, provided on the grip 26 and the housing part 27 respectively.

In the embodiment shown in fig. 2 and 3 the housing part 27 is provided with an annular groove 38 wherein an annular wall 39 of the grip 26 extends. Within the annular wall 39 a plunger element 40 is provided, which is hollow and has a lower end 41 extending within an inner wall 42 of the groove 38, and an upper end 43 extending above said inner wall 42. The lower end 41 is provided with an end wall 44 having a central opening 45. A seal 46 is provided in or on said opening 45. An upper end 47 of the grip 26 is provided with an upper end wall 48. The chamber 24 is substantially defined by the plunger element 40 and the upper end wall 48. A bottom part 49 of the housing part 27, is connecting to or partly forms the first coupling element 4. In the initial, first or maximum volume position as shown in fig. 2 a space 51 is provided between the lower end wall 44 of the plunger element 40 and the bottom part 49. In the space 51 a piercing element 52, such as a needle or spike is provided, with a sharp end 53 facing the opening 45 and directly adjacent the seal 46. The bottom part 49 comprises a channel 50 extending between the filter 12 and the space 51. The channel 50 widens above the element 17, for a wide distribution of fluid. At least one passage 54 is provided along the element 52, from the space 51 into the channel 50.

In the first, initial position the chamber 24 is completely filled with the fluid 21. Thus, if the grip 26 is brought towards the end part 49, the seal 46 is pressed against and over the piercing element 52, opening the chamber 24 towards the space 51. Fluid will flow from the reservoir 20 and into the channel 50. Moving the grip 26 further will first force the end wall 44 against the bottom part 49, limiting the movement of the plunger element 40. Further movement of the grip will then bring the upper end wall 47 further towards the bottom part 49, thus reducing the volume of the chamber 24 and therefore of the reservoir 20, forcing more fluid out of the chamber 24 and into the space 51 and channel 50 under controlled pressure. The fluid is forced into and through the element 17, through the filter 12 and the fluid connection 22, which can comprise or connect to channel 13, and into contact with the reagent 16. In the element 17 blood is diluted by the fluid, where after the combined fluid and blood are filtered by the filter 12, prior to coming into contact with the reagent 16. As a filtrate therefore the fluid with plasma will leave the filter. Since the grip has a fixed start position and a fixed end position, the amount of fluid leaving the chamber 24 is well defined, and therefore the dilution ratio is well defined.

In an embodiment the grip may be designed to rotate relative to the housing part in order to obtain a translation of at least the plunger element for reducing the volume of the reservoir, wherein during such rotation an interval can be provided in which the rotation does not provide for said translation or only to a very limited extend, especially directly after puncturing the seal 45, in order to allow pressure relief and/or pressure equalisation in the first part, especially over the seal 45, before further reduction of the volume by further rotation of the grip resulting in a further translation.

In the upper end wall of the chamber 24 a further opening 55 can be provided, closed off by a further seal 56. Through this opening the chamber can be filled, and/or additives can be added to the chamber 24 or fluids therein. This can be done prior to positioning the seal 56 over the opening 55, or by injecting such additives through the seal.

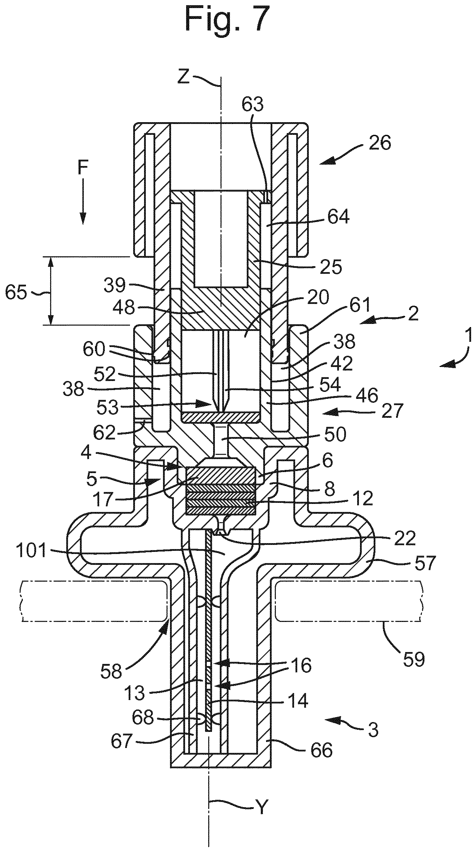

In fig. 7 an alternative embodiment of a device 1 of the invention is disclosed, wherein parts and features are referred to by the same or similar reference signs as used in fig. 1 - 6 for the same or similar parts or features. This embodiment shall only be described as far as it is substantially different from the first embodiment.

In fig. 7 the second part 3 has a longitudinal direction Y extending substantially parallel to the axis X of the first part 2. The strip 14 extends in a channel 13 extending in said longitudinal direction Y, from the filter 12 to at least the or each reagent 16. The second part 3 can be substantially cylindrical and can be provided with a flange 57 extending around the part 3, for supporting the second part 3, for example between two fingers of a hand or in an opening 58 in a support 59, shown in phantom lines in fig. 7, through which part of the second part 3 can pass but the flange 56 cannot. In this embodiment the grip 26 can be designed as described before with respect to fig. 2 - 6 or to be movable in a linear direction F only for reducing the volume of the reservoir 20. In the latter embodiment, which can also be used with the configuration of the second part 3 of fig. 1 - 6, the plunger 25 can be movable freely, wherein the fluid leaving the reservoir through a channel 50 having restricted cross section, can provide for a counter force on the plunger, in order to restrict its speed of movement. In the embodiment shown in fig. 7 the wall 39 of the grip can slide in the groove 38 of the housing part 27, wherein a sliding seal 60 is provided on both sides of the wall 39, against the inner and outer walls 42 and 61 of the groove 38, such that these are substantially air tight. Through the outer wall 61 near the end portion 49 of the housing part 27 a hole 62 is provided, having a small cross section, such that air trapped inside the groove 38 can only be expelled through said opening 62 with a restricted debit, such that again the speed with which the plunger 25 can be pressed towards the end position is restricted. The air in the groove 38 will act as an air cushion or spring element, providing a counter force on the plunger 25. A similar opening 63 could be provided in the grip 26, in order to aerate the space 64 within the grip 26 above the inner wall 42.

In the embodiment shown in fig. 7 a seal 46 is provided over the channel 50 on the side of the reservoir 20. Within the reservoir 20 the piercing element 52 is provided, connected to the plunger 25 and with the sharp end 53 facing the seal 46. Upon pressing the grip 26 with the plunger 25 down in the direction F the element 52 will be pierced through the seal 46 and into the channel 50. The mechanism 24 and/or the path of travel 65 between the starting and end positions of the plunger 25, for example defined by an upper end of the outer wall 61 of the housing part and a facing lower end (in a position as shown in fig. 7) of an outer wall part 66 of the grip 26, can be such that the end 53 will be stopped before reaching the element 17. The element 52 can have a cross section different from that of the channel 50, in size and/or shape, such that at least one passage 54 is created between the element 52 and the wall of the channel 50, for passage of fluid 21 there through or along the element 52 in the channel 50. In the embodiment shown the cross section can for example be in the shape of a plus-sign.

In the embodiment of fig. 7 the second part 3 can at least partly be made of a transparent material, such that at least the reagent or reagents 16 can be seen through the walls 66, 67 of the part 3, at least when there is a visual reaction between the reagent and the fluid flowing in the channel 13 and/or through the strip 14. In the embodiment shown the second part 3 comprises an outer wall 66 and an inner wall 67, the latter defining the channel 13. The strip 14 is held spaced apart from the wall 67 by spacer elements 68. In embodiments at least the outer wall 66 could be omitted.

It shall be clear that parts of the embodiments shown in fig. 1 - 7 could be combined into alternative embodiments, which combinations and permutations are considered to have been disclosed herein as well, as have the different parts as such individually. Especially the first and second parts 2, 3 as disclosed herein in combined devices 1 should also be considered to have been disclosed individually and/or as part of a kit-in-parts. In such kit-of-parts also a lancet or piercing or pricking element can be included for pricking a finger or such body part and drawing some blood to be adsorbed and/or absorbed in and/or on said element 17.

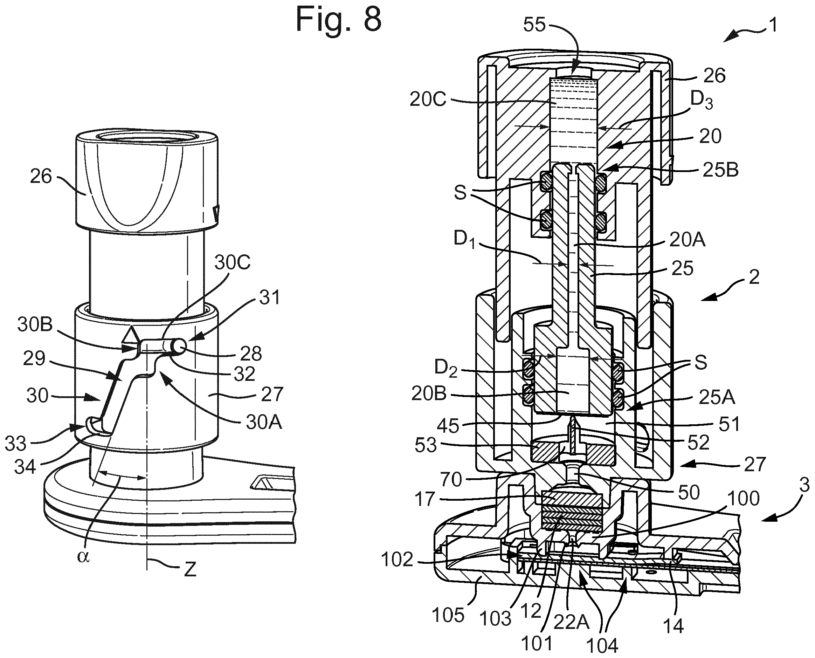

Fig. 8 - 10 show an alternative embodiment of a device 1 of the present disclosure. In the discussion of fig. 8 - 10 mainly the differences with the embodiments discussed before shall be discussed. The same or similar parts and functions shall be referred to by the same or similar reference signs. The embodiment mainly differs from the previous embodiments as discussed in the first part 2, in which mainly the grip and reservoir have been amended.

In the embodiment of fig. 8 - 10 the plunger 25 comprises a channel 20A having a relatively small cross section D1, which at an end 25A at the side of the chamber 51 can have a widened part 20B, for example having a cross section D2. In the grip 26 above an end 25B of the plunger 25 a part 20C of the reservoir 20 can be provided, which has a cross section D3. The channel 20A, the widened part 20B and the reservoir part 20C together effectively define the volume of the reservoir 20 when the device is in the first position. At the lower end 25A of the plunger the widened part 20B is closed off by a seal 45. In the grip 26 again a closed off opening 55 can be provided. The channel 20A preferably has a cross section D1 such that flow through said channel 20A is restricted. This can limit the flow of fluid to flow from the part 20C of the reservoir 20.

In this embodiment the grip 26 and the housing part 27 both comprise seals such as O-rings S extending around the plunger 25, for liquid tight sealing of the plunger relative to the housing part 27 and/or the grip 26. In embodiments other seals can be used, such as for example a luer lock in stead of the O-rings between the plunger and the housing part, in order to allow air to escape passed said seal. Again a piercing element 52 with a sharp end 53 is provided in the space 51. In the first position as shown in fig. 8 the sharp end 53 is in close proximity of the seal 45. The opening 70 around the element 52 and into the channel 50 are such that the speed of the fluid flowing through the channel 50 will not be too high.

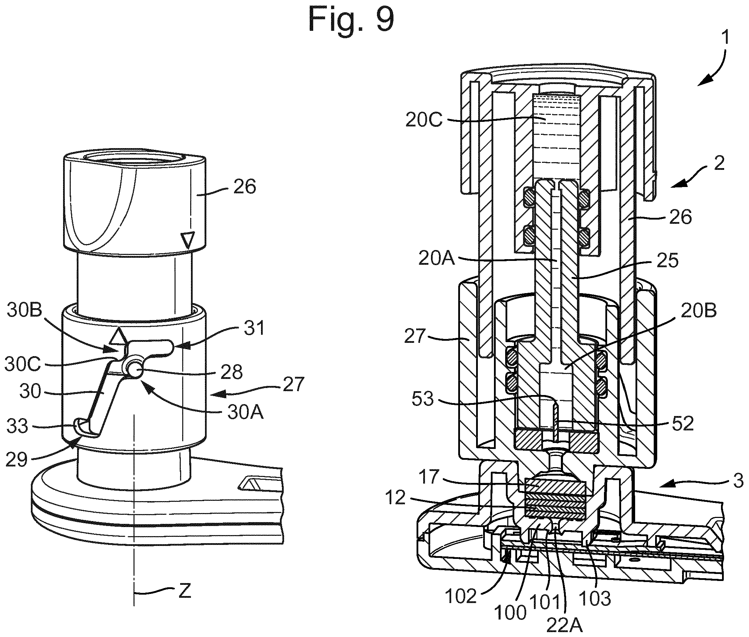

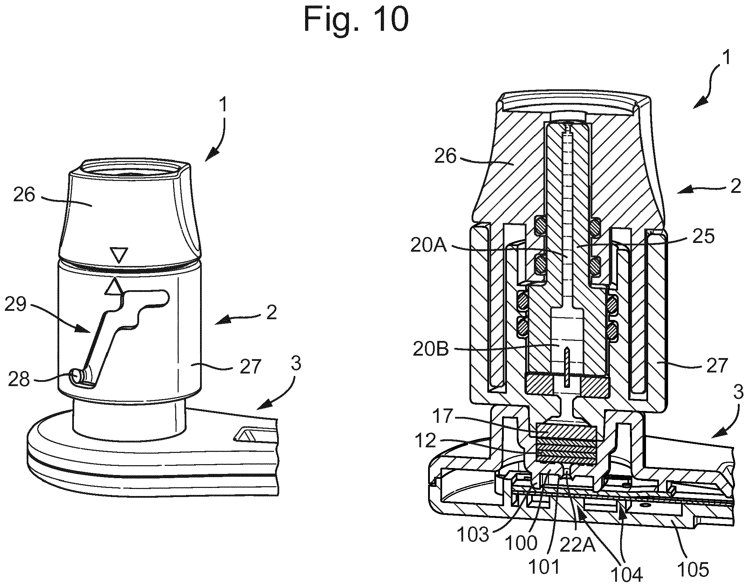

In this embodiment again the housing part 27 and the grip 26 are provided with at least one tooth 28 and a guide track 29 cooperating therewith. The guide track 29 again has a mid section 30 extending mostly at an angle α relative to the axis Z, such that is substantially spirals around part of the housing part and/or grip. Again the first and second ends 31, 33 can be provided with restrictions 32, 34 as discussed before, for the same purpose. In this embodiment between the first end 31 and the mid section 30 there is a portion 30A of the track 29 comprising a first part 30B extending substantially parallel to the axis Z adjacent the first end 31 and a second part 30C extending substantially perpendicularly to said axis Z, or at least at an angle to said first part 30B, between the first part 30B and the mid section 30. Again rotation of the grip 26 will eventually lead to a translation of the grip 26 and/of of the plunger 25 relative to the housing part 27, between the first position as shown in fig. 8 and comparable to fig. 2 and the second position as shown in fig. 10 and comparable to fig. 3, reducing the volume of the reservoir 20 and forcing a predetermined amount of fluid from the reservoir 20 through the element 17. In this embodiment however the at least one tooth 28 will have to pass the portion 30A before entering into the mid section 30. With the tooth 28 in the first part 30B, the grip 26 will translate in the direction Z over a distance equal to the length of said first part 30B, thus piercing the seal 45 by the element 52. Since the tooth 28 will at the end of part 30B engage a wall portion of the second part 30C extending at an angle relative to the first part 30B, as shown in fig. 9, said translation will be stopped, at least temporarily. This allows pressure equalisation and/or pressure relief in the reservoir and/or over the pierced seal 45. In this position the plunger 25 can be moved with it's lower end 25A against a bottom of the space 51.

From this position a further rotation of the grip 26 relative to the housing part 27 will move the tooth through the second part 30C and into the mid-section 30 for further translation of the grip 26 relative to the plunger 25 as described before. This will further reduce the volume of the reservoir 20 and push the predetermined amount of fluid out of the reservoir and through the element 17 and filter 12.

In this embodiment the channel 20A can act as a flow restriction for fluid flowing from the reservoir part 20C in the grip, which can add to restricting the possible speed of translation of the plunger and grip and thus of the flow with which the fluid is passed through the element 17 and into the filter 12. Moreover the channel 20A can ensure that the plunger 25 will first be pushed with the seal 45 over the element 52 and against the bottom of the space 51 before a significant amount of fluid is pressed from the reservoir part 20C into the channel 20A and through the element 17, thus preventing part of the fluid being trapped inside the space 51. A further effect of the channel 20A can be that it elongates the plunger 25 and grip 26 in order to provide a similar volume of the reservoir as is provided in the previously described embodiments, which can have the advantage that a longer translation is necessary for reaching the second position, compared to the previously described embodiments.

In embodiments of the disclosure the filter 12 can be a mono-body filter or can be a layered filter, different layers having different properties. In embodiments shown the filter 12 can have for example four layers, of which at least two outer layers 69 are wide pored support layers, and wherein at least one of the layers in between the outer layers can be a filter layer 70 having smaller pores, such that red blood cells cannot pass said filter layer having the smaller pores. The other layer 71 of the two in between layers can for example have wider pores than the filter layer 70 and the same or smaller that the outer layers 69, such that it can pre filter the fluid before entering the filter layer 70 having the smallest pores. In other embodiments a different number of layers and/or layers having different filtering and/or support properties can be used, depending for example on the nature of the sample to be used, the desired filtering capacity and the desired flow of fluid through the filter and/or the desired debit and pressure of the fluid on both sides of the filter 12. By way of example only a filter 12 in embodiments can be comprise two outer layers 69 made of fiber filter material having a pore size such that it retains particles larger than about 3 to 3.1 µm, a first intermediate filter layer 70 having a pore size for filtering out particles larger than about 1 pm and a second intermediate layer 71 having again a pore size for filtering out particles larger than about 3 to 3.1 µm. The filter material can by way of example be liquid filtration material such as binder-free glass filter paper as manufactured by Ahlstrom, USA, for example grade 141 for the outer layers 69 and the second intermediate layer 71, and a grade 121 for the first intermediate layer 70. These sizes and materials, as well as configuration of the filter 12 are only given by way of example and should not be understood as limiting the disclosure in any way.

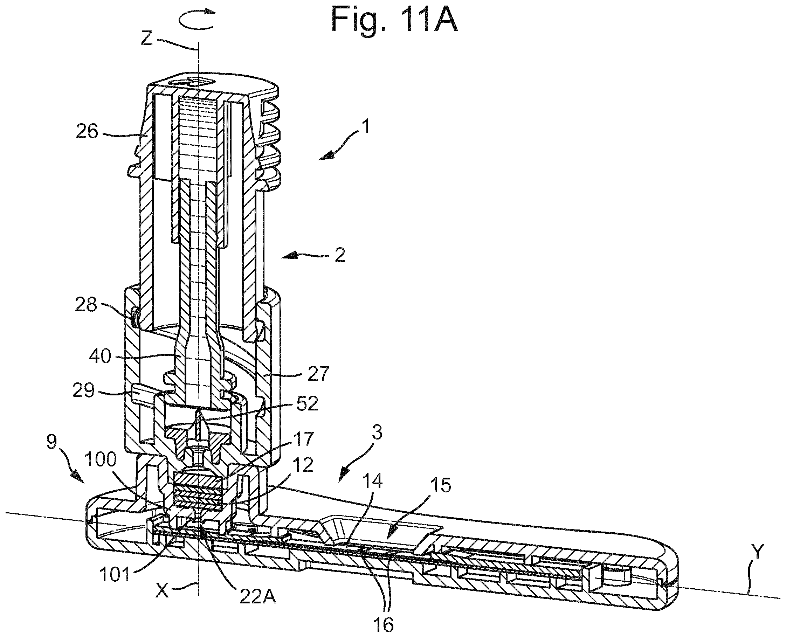

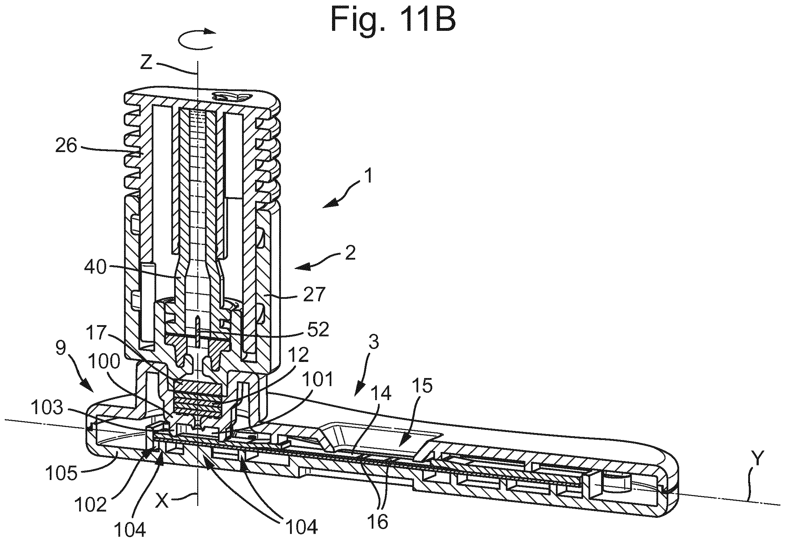

Fig 11a shows a further embodiment according to the invention, showing a device 1 where the first part 2 is placed on a second part 3 and where the devise is depicted in a first position of the first part 2. Figure 11b shows the device 1 in a second position of said first part 2, where the plunger has been pressed onto the piercing element 52. Here, the second part 3 is similar to or identical to the second part 3 as shown in figure 1. The grip 26 is again designed to be rotated about the axis Z via at least one tooth 28, extending radially outward from thee grip 26 engaging with a groove and/or track 29 at the inside of the housing part 27 of the second part 2. To bring the first part from the first to the second position, the grip 26 has to be rotated with 180 degrees or more, preferably 270 degrees or more, more preferably at least 360 degrees or more, such that the plunger 40 is moved between the first and second position. This will slow down the speed with which the plunger 40 will be moved on average between the first and second position, especially since the grip 26 will have to be released and regripped by a user before reaching the end position.

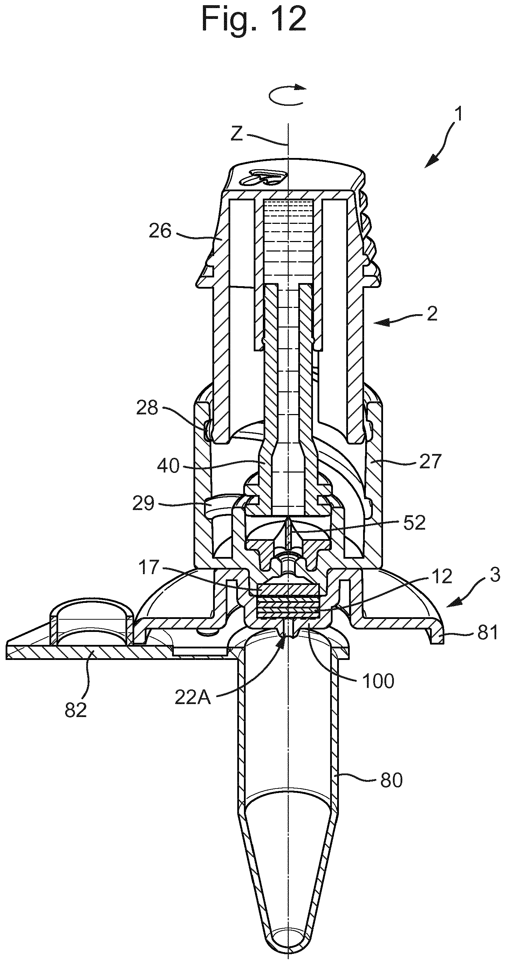

In all embodiments the second part can comprise or be formed by a receptacle for receiving the filtrate for storage and/or transport and/or treatment and/or testing in a different manner than by reagents, such as by radiation, illumination, viewing or otherwise. The filtrate can for example be cryo-stored in said receptacle 80. Fig. 12 shows an embodiment in which, by way of example only, the second part 3 is shown as a vial 80. In the example, the vial 80 comprises a holder part 81 for receiving the first part 2 in the same manor as described in the embodiments above. In the holder part 81 at least a filter 12 is provided. The holder part 81 is preferably provided with second coupling means and is preferably releasable from the vial 80, such that a lid 82 can be closed over the vial 80 and the holder part 81 can be discarded. The first part 2 can for example be a first part 2 corresponding to the first part 2 of figure 11. However, a different suitable first part 2 may be used as well, such as a first part of figure 2 or 3.

With a device and method of the present disclosure testing of samples can be performed faster and more accurate than with conventional methods and devices. Which may be of very high significance, especially when testing for irregularities that require instantaneous intervention when detected, such as life threatening health problems. With a device of the present disclosure the pressure exerted on the sample is well controlled, irrespective of the person performing the test, aiding in accurate and robust, dependable test results. The operation of the device is simple and intuitive, preventing mistakes in operation, which can be highly important, especially in life threatening situations. The device, especially the first part with the filter can be used with all kinds of test platforms known in the art, making it universally applicable. The dilution factor can be defined accurately and can easily be adapted, for example by defining a different volume of the reservoir, a different path of travel between the two extreme positions of the grip and/or by replacing the adsorbing and/or absorbing element 17 by a different element 17 having a different capacity. By for example adjusting the track, for example amending the angle of the mid section and/or design and positions of restrictions and/or portion 30A the ratio between rotation of the grip and the reduction of the volume of the reservoir can be adjusted, for adjusting the flow of fluid from the reservoir, as can be the channel portion in the plunger and/or other channels and/or openings of the device.

In an alternative embodiment the first part can be designed as previously described, wherein instead of a rotating grip the plunger 40 in the first position is biased towards the piercing element 52, for example by a spring as a biasing element, such that the seal 45 is close to the piercing element 52. A locking element can be provided for maintaining the plunger 40 in the first position. By releasing the locking element the plunger can be released, such that the biasing force of the biasing element is released and the plunger is forced forward, piercing the seal 5 on the piercing element 52 and then reducing the volume of the chamber and forcing the fluid through the element 17, similar to the previous embodiments. The biasing element preferably provides a substantially even force on the plunger towards the second end position, preferably such that the fluid is forced through and/or passed the element 17 and through the filter 12 with a substantially constant flow, sufficiently high to provide for a sufficiently quick test but without lysis of the blood cells.

In the embodiments having a grip 26 rotating in order to move the plunger forward towards the filter 12 and/or element 17, the track or tracks 29 extend over a sufficient length and angle to have to rotate the grip 26 relative to the housing part 27 over a substantial angle, preferably such an angle around the axis Z that a user has to reposition his or her hand during the rotation. This will further prevent a too high speed of the plunger and/or too quick a reduction of the volume of the chamber 24. The track or racks 29 extend preferably over at least 180 degrees, more preferably over at least an angle of more than 270 degrees around the axis Z. The angle over which the grip 26 has to be rotated around the axis Z can for example be about 360 degrees or more. This will slow down the speed with which the plunger will be moved on average between the end positions.

In a device as disclosed the filter 12 rests on or is provided above a wall 100 of the part 3, in which wall a channel part 22A of the channel 22 is provided. This channel part 22A, that is the channel 22 opens into a space 101 adjacent an end 102 of the test strip 14. Filtrate leaving the filter 12 will thus enter said space 102 through the channel 22 and engage the said end 102 of the strip 14 in said space. The end 102 of the strip 14 can be held in the second part 3 by being held between a wall 103 extending from the wall 100, and a support 104 provided at an opposite side of the strip end 102 in a bottom portion 105 of the second part 3. The wall 100 can provide ample support for the filter and keeps the filter separate from the test strip 14. The channel part 22A can concentrate the flow of filtrate into the space 101 and onto the test strip end 102.

In embodiments of a device 1 according to the present disclosure, for example as discussed with reference to fig. 1 - 12, but also in similar devices for testing bodily samples, such as especially blood sample, such as for example known from WO2009/139632, US2003/0175167 or US4477575, a sample absorbing and/or adsorbing element 17 can be provided with is provided with a reactive component, for example in and/or on the element 17, reactive with components of the sample and/or components on, in or of a test strip 14. Such reactive component can for example be a reagent, an agent, a conjugate or a blocking agent. Similarly or additionally such reactive components could be provided in and/or on the filter, if present in the device 1. A reactive component should at least be understood as a component with a component of the sample and/or a component in and/or on the test strip, which may include but is not limited to interacting chemically and/or physically with such sample component and/or binding to binding sites of such sample component.

In an embodiment the reactive component can be a reagent for determining the plasma concentration in a blood sample, that is the ration between plasma and cells in the sample, especially red blood cells. This can for example be used in recognising abnormalities in the ratio, which for men would normally be between about 49 - 59% plasma volume in a sample and for women between about 54 and 64%. This ratio can be used in assessing test results in and/or on the test strip. Such active compound can for example be any non human protein or compound, such as e.g. Glyceraldehyde 3-phosphate (G3P).

In an embodiment the reactive component can be an control agent for determining the dilution factor of the sample when mixed with the buffer, such that on the test strip or at least after filtering the dilution factor can be determined, such that the test results can, if necessary, be corrected for such dilution factor, such that the results can for example be standardised, for example be calculating hemacrotit (Hct) value. This can preferably be combined with the reagent for determining the plasma concentration as discussed here above.

In an embodiment the reactive component can be an anti coagulant, preventing coagulation of the blood sample when absorbed and/or adsorbed in and/or on the absorbing and/or adsorbing element. This can be advantageous when the testing, especially the filtering is not performed immediately after taking the sample. Such reactive component can for example be EDTA, heparin or citrate.

In an embodiment the reactive component can be an anti-lysis agent, suitable for preventing or at least reducing even further lysis of red blood cells of the sample, such that colouring red of the filtrate will be reduced or prevented.

In an embodiment the reactive component can be a conjugate for reaction with and/or binding to components of the sample, for example in blood or blood serum or plasma. Such components of the sample can for example be an acid base (AB) conjugate, proteins and the like. By providing such reactive components in and/or on absorbing and/or adsorbing element the incubation time for the (re)active components can be extended, which can improve the binding and/or effectiveness of the reaction. Such reactive component can for example be any antibody or antigen reacting the respective targets antigens of the antibodies to be determined. In examples as provided below such antibodies could for example be mouse-anti-H-FABP antibodies.

In an embodiment the reactive component can be a blocking agent, which can connect to and/or react with components of the sample, blocking the possibility of undesired (further) reaction between said component and an active component on the test strip, such as a reagent. The blocking agent can for example bind to binding positions of components of the sample, such that other components cannot later bind to such binding positions. In another example a blocking agent can bind to and/or react with components of the sample, such that these components can be filtered out by the filter or are otherwise prevented from reaching the test strip and/or a reactive component such as a reagent of said test strip. Such reactive component can for example be antibodies or antigens against targets cross reacting with antigen or antibodies to be determined, such as but not limited to against B-FABP, which is considered structurally most similar to H-FABP of all FABP's.

In an embodiment the reactive component can be an agglutination agent, such as for example wheat germ agglutinin (WGA) for agglutinating red blood cells, for example before filtering. Filtration of larger agglutinated red cells may allow the use of filters with larger pore size that may have lesser resistance. This will lower the pressure and thus (risk of) lysis and allow faster filtration to reduce filtration time.

In an embodiment the reactive component can be a lysing agent, such as tris-HCI, EGTA, SDS, triton-X or similar known lysis agents. In such embodiments the filter may be omitted or used for filtering out other components of the sample, for example after binding such components by an active component provided prior to filtering. Such lysing agent can be advantageous when testing for example DNA.

By providing an active component in and/or on the absorbing and/or adsorbing element the active component can be contacted and interact with the sample at an early stage, ensuring relatively long contact with the sample, compared to when providing such component downstream of the filter. This can provide for a suitably extended incubation time. By providing an active component in and/or on the absorbing and/or adsorbing element the active component can contact the sample before filtering, such that the active component can interact with components of the sample which may be filtered out, such as red and/or white blood cells. By providing an active component in and/or on the absorbing and/or adsorbing element the active component can be provided in a dry form, for example separated from the liquid buffer, such that it can for example be preserved for an extended period of time. The active component or components can for example be dried, such as but not limited to freeze dried. By providing an active component in and/or on the absorbing and/or adsorbing element, any number of active components can be provided, for example active components which are non-reactive with each other on the element, or can be provided in separated positions in and/or on the element. By providing an active component in and/or on the absorbing and/or adsorbing element the device 1 can easily be adopted for a specific test by exchanging or providing a test specific element to the device, which can be an otherwise standard device. Similarly and/or additionally such active components can be added to a filter.

The or at least one such reactive component provided in and/or on the absorbing and/or adsorbing element can alternatively and/or additionally be reactive with a component provided in the buffer and/or in and/or on the filter.

Different reactive components can be combined in a device, especially in and/or on an absorbing and/or adsorbing element. Absorbing and/or adsorbing element should be understood at least as including elements which can hold a fluid sample, such as a sponge like element, and can also include capillary elements, vials and the like.

A device or kit of parts according to the disclosure can be provided with different absorbing and/or adsorbing elements, provided with different reactive components, such that the device or kit can be used for different tests, depending on at least the absorbing and/or adsorbing element. In such kit for example one absorbing and/or adsorbing element can be provided with an anti coagulate as reactive component, to be used when the test is not performed instantaneously, and a second absorbing and/or adsorbing element without such anti coagulate which can be used for immediate testing. In a kit for example one absorbing and/or adsorbing element can be provided with and one without a blocking agent, such that the same test strip can be used for two different tests, depending on which absorbing and/or adsorbing element is used.

Hereafter experiments will be described, elucidating use of the devices, kits of-parts and methods according to the description.

A device of the present invention can be provided with a reagent for detection, i.e. for the determination of the quantitative, semi-quantitative or qualitative presence of an analyte such as a chemical or biological substance or microorganisms in blood plasma or any other filtrate obtained with the first part, for example but not limited to human or animal excrements such as urine, faeces, saliva, body tissue or the like. The reagent can be provided in any suitable position and form, as long as it will be brought into contact with the plasma and/or filtrate such that a discernible reaction between the reagent and the filtrate can be obtained if a reactant to said reagent is present in the filtrate in a detectable amount. It may be on the strip as discussed, but can also be or in stead of be provided entirely or in part in the fluid, in the element 17, in the filter 12, on a wall of the channel 13 or combinations thereof. Also reagents can be used that are multi component reagents, such that only a reaction can occur if they are combined in the filtrate.

For instance, a reagent can be used which may indicate the presence of antibodies against foreign antigens (organism) for example Helicobacter pylori or native antigens that indicate a pathological state or disorder.

Also reagents can be used that indicate the presence of antigens such as an extent in which they must be present or that a limit is exceeded, for example antigens by which the presence of tumors can be demonstrated or can be made credible , or that indicates an excess or deficiency of coagulation factors.

Reagents can also be applied with which the presence of, for example, vitamins can be determined.

Reagents can further be used with which by an overshooting or undercutting of a threshold or limit it can be indicated whether the overshoot is detrimental to the patient. Such reagents can advantageous be combined with a reagent that indicates overshooting or undercutting of that limit. Furthermore reagents can be applied with which a therapeutic blood level of a substance can be determined, for example a drug or toxin, such as a drug which, for optimal functioning, depends on an optimal blood level that may not be exceeded because of, for instance, undesirable side effects. Also combinations of reagents as mentioned can be applied. These reagents and applications are of course only illustrative, and should not be interpreted restrictively.

In the present description, the term "reagent" or "reagents" or similar wording should at least be understood to include antibodies or enzymes, which means that tests can be applied that are antibody or enzyme-based.

Several reagents and other markers can be applied, such as antigens, chemical reagents, enzymes, chemical markers and the like. Reagents and markers could be used to indicate problems with heart, liver, kidney or other organs, glucose abnormalities such as diabetes, cholesterol disorders, defects in one or more hormones or cytokines, diagnostic parameters in general and such, viral or bacterial abnormalities such as influenza, malaria, hepatitis, HIV, inflammation, MS, ME, and other indicators, especially for existing and/or potential health problems. Deviations in this context should be understood as such deviations from normal values of which it can be expected that, for the patient at issue, these values indicate or should indicate to a physician that further investigation or intervention is needed by, for example, administration of drugs, fluids, or nutrients or by surgical intervention.

Examples of reagents, which are by no means limiting the invention, include for example antibodies for viruses, for example HTLV I and/or II, Influenza, or markers for organ functions, such as cardiac or cardiovascular problems, heart attacks (myocardial infarction) and/or stroke, monoclonal antibodies, coagulation reagents such as lupus anticoagulant sensitive or insensitive reagents, PSA antigen, HBS-1, HLA antibodies, HbA(1c) or GlyHb in hemoglobin measurement.

In a first example of an embodiment of a reagent 16, 2 H-FABP antibodies (Hycult), which are suitable for the demonstration of H-FABP were applied on the strip 14. In the reservoir 20 a quantity of diluent (buffer) was provided (e.g. 300 microliters) as said fluid 21. An amount of blood was absorbed by the element 17, eg. 30 microliters of blood. By movement of the grip 26 and therefore of the plunger element 40, 180 microliter of buffer was forced through the element and filter resulting in 75 microliter diluted plasma, which was analyzed by the strip 14 (effectively diluting the bloodplasma about 7 times) This plasma was brought into contact with the reagents 16, as a result of which the reagent H-FABP complex accumulated on the strip and discoloured from a neutral colour to a distinctive colour, in this case red, clearly visible from the outside through the widow. By this, it was found that the H-FABP level in the blood was higher than a threshold of 4,4 ng/ml. Control measurements of whole blood drawn by venous collection and tested in a laboratory showed that the blood indeed had a value above that threshold.

An embodiment of special preference for the detection of markers for kidney function, liver, and/or stroke will be described below.

This embodiment of special preferences can in particular be applied to the quantitative, semi-quantitative or qualitative detection of FABP in samples of body tissue or body fluids. Fatty acid-binding protein (FABP) is a protein known as an early marker for damage to specific tissues wherein each tissue type is characterized by its own FABP type. FABP are 15 kDa cytosolic proteins involved in intracellular binding of fatty acids and are expressed in nine different isoforms, each named after the tissue in which it was first described.
- Heart-FABP (H-FABP or heart-type)
- Liver-FABP (L FABP or liver-type);
- Intestinal-FABP (I-FABP or small intestine type),
- Ileal-FABP (ILBP or ileum-type)
- Brain-FABP (B-FABP or brain-type)
- Adipocyte-FABP (A-FABP or fat cell-type)
- Epithelial / epidermal-FABP (E-FABP or epithelial cell-type),
- Testicular-FABP (T-FABP or testicular-type) and
- Myelin-FABP (M-FABP or nerve cell-type).

To date, there are no rapid tests for FABP that can give sufficiently accurate results in less than a five to six minutes. The rapid determination of FABP may lead to a rapid diagnosis of tissue damage and early commencement with the proper therapy in particular in life threatening conditions such as heart disease or stroke. Measurements on the quantity of specific FABPs may, amongst others, but not exclusively, be applied for the diagnosis of myocardial injury (H-FABP), liver damage (L-FABP), kidney damage (L-FABP and/or H-FABP), intestinal damage (including I-FABP, ILBP and/or L-FABP), brain damage (B-FABP and/or H-FABP), in the diagnosis of a series of disorders to the lipid metabolism, diabetes, inflammatory disorders, multiple sclerosis, atherosclerosis, cancer and tissue rejection after transplantation and as prognostic indicator and/or risk stratification in patients with AMI, acute pulmonary lung embolism and/or severe sepsis and/or septic shock.

The present invention provides for the detection of basically any of the above FABPs in each isoform in which it can occur in an animal or human patient, wherein the detection, in connection with the desired specificity, is preferably performed on the basis of an immunoassay, and preferably in blood.

Immunoassays for the detection of FABP are in principle known to the skilled person, and such assays are suitable for use in the present invention. An example of an available immunoassay takes the form of a sandwich ELISA (Pelser MMAL. 2004. "Fatty acid-binding protein as plasma marker for tissue injury." Thesis University of Maastricht, Netherlands ISBN 90-9018161-X, Chapter 3, p. 43-51; Wodzig KWH, Pelser MMAL, van der Vusse GJ, Roos W, Glatz JFC. One-step enzyme-linked immunosorbent assay (ELISA) for plasma fatty acid-binding protein. Ann Clin Biochem 1997; 34:263 - 8). This assay, with a total duration of 45 minutes, is the fastest, sufficiently specific and sensitive H-FABP ELISA which is commercially (Hycult Biotechnology) available. This assay makes use of two different monoclonal antibodies, each directed to a different epitope of H-FABP. One of these monoclonal antibodies acts as capture-antibody and is attached / immobilized to a detection surface. The other antibody is conjugated with horseradish peroxidase (HRP) and serves as detection-antibody. The monoclonal antibodies which are applied in this assay are described in more detail elsewhere (vide Pelser MMAL. 2004, supra Chapter 3, p. 43-51 and Chapter 4, p. 53-67; Roos W, Eymann E, M Symannek, Duppenthaler J , Wodzig KWH, Pelser MMAL, Glatz JFC. "Monoclonal antibodies to human heart fatty acid-binding protein." J Immunol Methods 1995; 183:149-53). The detection may, after formation of a capture-antibody / H-FABP / detection-antibody complex, be detected by using a HRP-specific enzyme substrate, such as the chromogen tetramethyl benzidine (TMB) which after conversion by HRP provides a blue reaction product which can be detected spectrophotometrically by measuring the absorption at 450 nm.

The skilled person will understand that many variations on the above detection principal can be used in aspects of the present invention.

Thus, antibodies against other FABP types then H-FABP can be used to detect other isoforms of this protein. Also other epitopes can be used for the binding of the antibody to FABP. The development of antibodies against other epitopes of a particular FABP for which an antibody to an epitope is already available, or the development of antibodies that exhibit specific binding with other FABP isoforms is within the reach of the skilled person and need not be described in detail here.

An antibody that can be applied as a reagent in aspects of the present invention can be a polyclonal or a monoclonal antibody. Preferably monoclonal antibodies are used. Antibodies can include complete immunoglobulins or a fragment thereof, wherein immunoglobulins can be selected from the different classes and isotype, such as IgA, IgD, IgE, lgG1, IgG2a, lgG2b and lgG3, IgM, etc. Fragments thereof may comprise Fab, Fv and F(ab')2, Fab', and the like. Furthermore, aggregates, polymers, and conjugates of immunoglobulins or fragments thereof can suitably be applied as long as the binding affinity for a given FABP is maintained.

The element which is commonly referred to herein as reagent 16 will usually be formed by the capture-antibody. This capture-antibody can be affixed to the surface of the strip and/or therein, such that the capture-antibody is or can be in direct contact with blood plasma. The strip 14 can fill the complete channel 13 or only part of it. Alternatively the strip 14 can be left out, such that the channel 13 in the second part 3 is open, wherein the reagent 16 can be adhered to the wall of the channel 13 or can be provided in for example fluid or solid form inside the channel 13. Adherence of the capture-antibody to a solid surface, for example, can be achieved through a biotin-(strept)avidin link. The capture-antibody can optionally be provided with a paramagnetic label so that it can be collected from a liquid and immobilized to a solid phase at any time during the reaction by magnetic attraction.

Preferably the reagent-bearing strip by its porous nature supports the uptake of diluted plasma after passing the filter 12. Porous can be understood as including but not limited to having capillary channels or pores for transporting fluid such as diluted plasma. Preferably, the reagent-bearing strip 14 is capable of taking up much or even most of the diluted, separated plasma. The FABP present in the liquid phase is preferably present in a form wherein it is complexed with a detection-antibody. It is highly preferred that the reagent-bearing strip 14 supports a capillary flow, whereby the plasma is drawn into the porous reagent-bearing element 14 under the influence of capillary force as a result of which it is brought into contact with immobilized reagent (i.e. immobilized capture-antibody). The plasma can for example in part flow under the strip and be sucked into the strip by said capillary force.

Preferably, the FABP test of the present invention is in the form of a LFIA (Lateral Flow Immuno Assay) or sandwich ELISA, wherein further a detection-antibody is used for the detection of the binding of the FABP to the capture-antibody. The binding of the detection-antibody to FABP may in principle occur prior to, during or after the binding of FABP to the capture-antibody. Preferably, it is first allowed that the detection-antibody binds to an FABP present in the body sample and then this complex is allowed to bind to the immobilized or to-be immobilized capture-antibody. In order to achieve this, the detection-antibody can be very suitably added to the diluent fluid in chamber 24 of the device 1 of the invention. In an alternative embodiment, the antibody can be added to the (preferably porous) element 17 with which the known quantity of blood is adsorbed and/or absorbed. Such an element 17 may take the form of a sponge, wherein the detection-antibody is present so that it can mix directly with the sampled blood, before the diluent fluid is forced through the element 17.

In another alternative embodiment, the antibody can be added to the filter 12 or in the channel 13 or preferably to the strip 14. It is important that the detection-antibody is mixed homogeneously with the blood or blood plasma under conditions in which binding to FABP occurs or is possible. In an embodiment wherein a capture-antibody is applied that is to be immobilized and which is first allowed to react with FABP in the liquid phase, this capture-antibody can be added to the diluent, to a blood drawing element or blood collection element or to plasma after separation of plasma by filter 12. In fact, every possible combination or sequence is possible, as long as the end result provides an immobilized complex of capture-antibody-/-FABP-/-detection-antibody. Obviously also other Elisa principles can be used, for example not of a sandwich type, in which only one Ab is used.

The detection-antibody may be labelled with any appropriate detection label, such as colloidal gold or silver, latex (or carbon), streptavidin, biotin, microspheres, latex beads, peroxidase, streptavidin-labeled horse radish peroxidase (HRP), phosphatase, alkaline phosphatase (AP) chromogenic labels, fluorescent labels, phosphorescent labels, chemiluminescent labels, secondary antibodies or any other suitable label with which detection of successful binding can be established. An optional secondary antibody may comprise any of the above labels.

Preferably, colloidal gold is used, because no washing steps are needed and a simple one-step test is obtained. Colloidal gold consists of discrete particles with a diameter of 10 nm to 100 nm and a very high extinction coefficient. When concentrated at a solid surface colloidal gold can very easily be observed visually as a red colour. Preferably, the capture-antibody is therefore applied in a recognizable pattern to preferably the surface of any element of the device 1 such as the strip 14 so that the capture-antibody is in contact with blood plasma and which surface can be observed at least in part from the outside of the device.

The skilled person will understand that at least lateral flow applications are envisioned in the present invention. The skilled person will also understand that alongside and parallel to the primary test a secondary test can be performed with which either a second FABP is detected, or by which a control-reaction is provided. To this end for example a second reagent can be applied in the same or similar manner as the first reagent. In other applications the filter 12 can be applied directly below the first part and the filtrate can be received in a different receptacle, such as but not limited to a receptacle for storage and/or shipment of the filtrate for later reference. This can for example be a vial, container, adsorbing and/or absorbing element or other suitable means.

Body samples which can be used in a test in accordance with the invention are in principle not limited to blood. Also other body samples such as tissue samples, or a sample of urine, faeces, saliva, tear fluid, mucus, sputum, semen, cervical secretions, cerebrospinal fluid, vomit, nasal secretions, sweat, amnion fluid, or breast milk can be tested.

In an example, the present disclosure provides a kit of parts, the components of which are preferably packed together. The kit preferably includes:
- A first part with diluent (such as first part 2 of device 1, according to the invention, comprising a chamber 24 with a diluent 21 as described above, and preferably with first coupling element or elements, as shown in the figures);
- A second part with a test strip (such as second part 3 of device 1, according to the invention, comprising channel 13 with reagent 16 and second coupling element or elements, as shown in the figures);
- A filter for filtering blood cells from a blood sample diluted with the diluent, (such as a filter 12) which may be provided in the first or second part, or as separate element for coupling with one of these parts) through which at least blood plasma can pass but through which red blood cells can not pass;
- An adsorbent and/or absorbent element which provides for the possibility of sampling a known quantity of blood (such as a sponge like element 17) which may be provided in the first or second part, or as separate element for coupling with one of these parts). This element can be applied to introduce a known quantity of a blood sample into the device 1 (typically about 30 or 60 pL of blood, but this amount may vary), whereby the blood constituents can be diluted by the diluent passed there through.

The kit of parts can further comprise:
- A capture-antibody that can bind specifically to a first epitope of FABP (reagent 16). The capture-antibody may advantageously be in a form in which it is immobilized, in or on a surface of a channel 13 or strip 14 that can be observed from the outside of the device 1. An antibody suitable for use as capture-antibody for the detection of H-FABP is anti-human monoclonal antibody H-FABP 67D3 (such as available from Hycult Biotechnology BV, Uden, Netherlands). A suitable surface is a porous part having capillary action, which can for example be applied in lateral flow detection;
- Optionally a detection-antibody that can bind specifically with a second epitope of FABP, which is different from the first epitope and wherein both antibodies do not materially affect each other's binding to FABP in a detrimental manner. The detection-antibody may be provided in the diluent. An appropriate concentration of a detection antibody in the diluent is 5 to 20 pg/L. The detection antibody could also be provided in the second part, for example in or on the strip 14. An antibody suitable for use as detection-antibody for the detection of H-FABP is anti-human monoclonal antibody H-FABP 66E2, (such as available from Hycult Biotechnology BV, Uden, Netherlands).

In a kit of parts the element 17 can be provided with one of more reactive components, as discussed before. In examples of the experiments Glyceraldehyde 3-phosphate could be added to the element 17, allowing assessment of the plasma/sample volume ratio, and for determination of the Hct value of the sample.

The application of the device and the detection system provides a point-of-care (POC) rapid test, ie a rapid test for use by general practitioners, in an ambulance, in a hospital or as a home-test for detection of H-FABP in plasma.

A method of detecting FABP in a sample of blood from a patient preferably includes the following steps:
- providing a device of the present invention as described above;
- introducing a known quantity of blood in said adsorbing and/or absorbing element;
- forcing diluent through said element, diluting the blood sample;
- separating (red) blood cells from blood plasma by forcing said diluted blood sample partly through said filter;
- promoting a reaction between blood plasma having passed said filter and a reagent. In embodiments this can mean contacting FABP in said blood or blood plasma with at least one of said detection-antibody and capture-antibody under conditions wherein specific binding occurs between the antibody and the FABP; and
- detecting the specific binding.

The different variations in examples on this process are explained in detail above.

The present invention will now be illustrated by the following examples which are in no way limiting the invention.

### EXAMPLE

Developing a point-of-care (POC) rapid test (a test for use in general practitioners office, ambulance, hospital or as home test for detection of H-FABP in plasma)

The test provides for the immunochemical determination of the presence of an increased concentration. Tests were designed for detecting > 4 pg/L of heart-type fatty acid-binding protein (H-FABP) in plasma, and were combined with a device as described herein. The time between the taking of the blood sample and obtaining a positive test result can be less than 60 seconds

The present example describes in detail a kit-of-parts as described above and as envisioned by the inventors. This embodiment includes:
- A reservoir comprising a diluent (for example 50 mM Sodium Phosphate, 0.137 M Sodium Chloride, 0.0027 M Potassium Chloride, 0.05% Tween-20, 0.05% Proclin 300, pH 6
- A sponge for collecting a defined amount of blood;
- A blood filter for the separation of plasma and blood cells
- A test strip for the detection of H-FABP in the filtrate.

The kit of parts may further comprise a lancet (finger pricker) for a cut in a fingertip on order for providing a blood sample or other means for drawing blood or obtaining a sample.

The sponge is applied against the cut in the finger or otherwise applied to introduce approximately 30 pL of blood from a blood sample into it. Diluent from the reservoir of the device 1 is forced through the element, such that the blood is diluted. The pressure with which the diluent is passed through the element and a relatively large surface over which the diluent is brought into contact with the element can contribute to dissolving the blood in the diluent

Hereafter the diluted blood sample is forced through a filter. The diluted blood is pressed through the filter with force, so that blood is pressed through the blood filter while the blood cells are arrested and remain in the element 17 and/or filter 12. The blood plasma is collected at the other side of the blood filter in the channel 13 of the second part. FABP present in the blood sample will essentially instantaneously bind to the gold-conjugated monoclonal antibody mAbdetect present in the first part (conjugate pad) of the strip to form a FABP-mAbdetect-gold complex.

At a distance of for example about 20 to 40 mm from the filter the porous strip comprises immobilized thereto a quantity ca. 200 ng of monoclonal antibody 67D3 (Hycult Biotechnology BV, Uden, Netherlands), directed against human heart-FABP type (which is referred to as the 'second antibody', mAbcapture) and which antibody recognizes an epitope on human heart-type FABP that is different from that recognized by mAbdetect.

Similarly, the porous part at a distance of about 20 to 40 mm from the filter comprises immobilized thereto a monoclonal antibody directed against another protein (for example, a control or reference or second test protein). The total length and volume (i.e. the size) of the porous part is preferably such that a significant portion (about 60 pL) of the diluted plasma sample is absorbed in the porous part.

After absorption of the plasma into the porous part the FABP-mAbdetect-gold complex will bind to the second monoclonal antibody mAbcapture immobilized thereon under the formation of a coloured band that is visible through the transparent or open wall of the stem. The intensity of this coloured band will increase with the concentration of FABP in the blood sample. In the event the FABP concentration in the original blood sample is < 4,5 pg/L, no coloured band will be visible.

Similarly, another protein that is present in the blood will bind to the specific antibody that is immobilized on the porous section at a distance of 10 mm from the lower end of the stem as described above, and if the diluent is also provided with a gold-conjugated antibody against another epitope of that protein, this complex will be visible as a coloured band on the porous part at a distance of 10 mm from the lower end of the stem. This reaction can be used as a control on the presence of blood plasma in the test and thus a proper implementation. Another method for providing a control is to apply an antimouse Ab as a control reactanton the strip 14. A free mAb detect was detected in order to check whether the conjugate mAb detect that was used for detecting H-FABP was still intact, in order to rule out false negative test results.

The invention is by no means limited to the examples of devices and methods described herein. Many amendments and variations are possible within the scope of the invention as defined by the claims. For example combinations of parts and devices as described are considered to have been disclosed herein too. The grip can be designed to translate, wherein the at least one tooth and track are designed to make the plunger rotate for obtaining a translation thereof for reducing the volume of the reservoir and displacing the fluid. The reservoir could be divided into two or more chambers, separated by pierceable seals or the like, each for containing at least one component of a multi component fluid, such that upon translation of the plunger the seals are pierced and the components are mixed.

## Claims

1. Test device (1) for testing a human or animal sample, such as blood or blood components (B), comprising a first part (2) and a second part (3), comprising co-operating first and second coupling elements (4, 5) for coupling the first part (2) with the second part (3), wherein the first part (2) comprises a fluid reservoir (20), and wherein the second part (3) comprises at least one receptacle, **characterized in that** a sample absorbing and/or adsorbing element (17) is provided which is fixed in the first part or can be introduced into the first part, wherein the fluid reservoir (20) is reducible in volume for forcing a liquid contained therein through and/or along the adsorbing and/or absorbing element (17), forcing the sample such as blood or components (B) thereof from the adsorbing and/or absorbing element (17) into the receptacle, preferably into contact with at least one reagent in the second part (3) when the first and second part (2, 3) are connected or brought into connection with each other by at least the first and second coupling elements (4, 5).

2. Test device according to claim 1, wherein the first part (2) is provided with the first coupling elements (4) at or near a first end (7) thereof, wherein the adsorbing and/or absorbing element (17) is provided at or near said first end (7), and wherein the fluid reservoir (20) is provided at a side of the adsorbing and/or absorbing element (17) opposite the first end (7).

3. Test device according to claim 1 or 2, wherein the first and second coupling elements (4, 5) are designed for releasable positioning of the first part (2) relative to the second part (3), such that a first end (7) of the first part (2) is positioned over an inlet of a channel (13) in the second part (3), wherein in the channel (13) at least one filter (12) is provided in the inlet or between the inlet and the at least one reagent (16).

4. Test device according to any one of the previous claims, wherein the fluid reservoir (20) comprises a chamber (24) and a plunger (25), movable relative to said chamber (24) for reducing the volume of the reservoir (20), wherein an outlet is provided for the reservoir (20), opening toward the at least one adsorbing and/or absorbing element (17).

5. Test device according to claim 4, wherein the first part (2) is provided with a grip (26), rotatable on or around a housing part (27) of the first part (2) comprising at least the chamber (24)and/or the plunger (25), such that a rotation of said grip (26) results in a movement of the plunger (25) relative to the chamber (24), for reducing said volume.

6. Test device according to claim 5, wherein the grip (26) is provided with at least one tooth (28) and the housing part (27) with a guide track (29) for said tooth (28) or vice versa, wherein the guide track (29) extends such that a rotation of the grip (26) leads to a forced movement of the grip (26) in a linear direction, preferably a movement towards or away from the adsorbing and/or absorbing element (17).

7. Test device according to any one of the previous claims, wherein the second part (3) comprises a test strip (14), preferably having a longitudinal direction, wherein the at least one reagent (16) is provided in or on the strip (14) and wherein the strip (14) is at least partly visible from outside the second part (3), preferably at least a part of the strip (14) comprising the at least one reagent (16).

8. Test device according to any one of the previous claims, wherein the first part (2) has a first longitudinal direction and the second part (3) as a second longitudinal direction, wherein the first and second longitudinal directions extend at a mutual angle different from 0 or 180 degrees and preferably substantially 90 degrees.

9. Test device according to claim 8, wherein the first part (2) has a first coupling element (4), comprising or formed by a substantially cylindrical part, whereas the second part (3) has a second coupling element (5) comprising an at least partly circular wall, extending substantially perpendicular to the second longitudinal direction, forming an opening for forming a lock, preferably at least a form lock and/or press fit lock, with the first coupling element (4), such that the first coupling element (4) can be inserted into the second coupling element (5) in the first longitudinal direction for coupling the first (2) with the second part (3).

10. Test device according to any one of the previous claims, wherein the first part (2) comprises a seal (46) sealing off the reservoir (20), wherein the first part (2) is provided with an element (52) for piercing or rupturing the seal (46), such that a fluid connection is formed between the reservoir (20) and the adsorbing and/or absorbing element (17).

11. Test device according to any one of the previous claims, wherein the first and second part (2, 3) are coupled by the first and second coupling elements (4, 5), wherein with the reservoir (20) in a maximally reduced volume position a fluid connection is provided between the reservoir (20) and the at least one reagent (16) through the adsorbing and/or absorbing element (17) and a filter (12) provided between the element (17) and the at least one reagent (16), wherein the fluid connection is substantially entirely filled with a fluid from the reservoir (20) and sample components contained therein, preferably at least blood plasma, whereas the filter (12) is preferably a filter suitable for filtering at least blood cells from the blood prior to contact with the at least one reagent (16).

12. Test device according to any one of the previous claims, wherein a filter (12) is provided for filtering at least blood cells from blood or particles from a sample (B), which filter (12) is a single, double or multi-layer filter.

13. Test device according to any one of the previous claims, wherein the first part (2) comprises a mechanism for opening the reservoir (20) and controlled reduction of the volume of the reservoir (20) for forcing liquid from the reservoir (20).

14. Test device, preferably according to any one of the preceding claims, comprising a blood absorbing and/or adsorbing element (17), for taking a blood sample (B), and preferably a filter (12) for filtering out elements from the sample (B), wherein at least one of the blood absorbing and/or adsorbing element (17) and the filter (12) is provided with a reactive component reactive with components of the sample (B) and/or components on, in or of a test strip (14).

15. Method of testing human or animal samples, such as blood or components thereof, wherein a quantity of a sample (B) is adsorbed and/or absorbed in and/or on an element (17), which at one side is connected to a reservoir (20) containing a liquid, especially a solvent or dilutant, wherein the element (17) is connected to a channel (13) comprising at least one reagent (16) and a filter (12), wherein when a first and a second part, comprising co-operating first and second coupling elements for coupling the first part with the second part and the second part comprises the channel, are connected or brought into connection with each other by at least the first and second coupling elements, the reservoir (20) is reduced in volume, such that the liquid contained therein is at least partly forced through and/or along the element (17), dissolving and/or diluting the sample (B) provided therein and/or thereon, and the then diluted and/or dissolved sample (B) at least partly through the filter (12), wherein in the filter (12) at least cells are filtered out, preferably at least blood cells, whereas the part of the diluted and/or dissolved sample (B) passing through the filter (12) is at least partly brought into contact with the at least one reagent (16).

16. Method according to claim 15, wherein the element (17) for adsorbing and/or absorbing blood is contained in a first part (2) of a test device (1), next to the reservoir (20), which element (17) is substantially saturated with blood, where after the first part (2) is connected to a second part (3) of the device (1), comprising the at least one reagent (16), where after the reservoir (20) is reduced in volume, forcing the liquid from the reservoir (20) through the element (17) and into and through the second part (3) into contact with the at least one reagent (16).

17. Method for preparing a human or animal sample for testing, wherein a quantity of a sample (B) is adsorbed and/or absorbed in and/or on an element (17), which at one side is connected to a reservoir (20) containing a liquid, especially a solvent or dilutant, wherein the element (17) is connected to a channel (13) comprising at least one reagent (16) and a filter (12), wherein, when a first and a second part, comprising co-operating first and second coupling elements for coupling the first part with the second part and the second part comprises the channel, are connected or brought into connection with each other by at least the first and second coupling elements, the reservoir (20) is reduced in volume, such that the liquid contained therein is at least partly forced through and/or along the element (17), dissolving and/or diluting the sample (B) provided therein and/or thereon, and the then diluted and/or dissolved sample (B) at least partly through the filter (12), wherein in the filter (12) at least cells are filtered out, preferably at least blood cells, whereas the part of the diluted and/or dissolved sample (B) passing through the filter (12) is received in a receptacle for storage and/or transport.

18. Kit of parts for a test device according to anyone of claims 1-14 or for performing a method according to anyone of claims 15-17, comprising:
- A first part (2) with a diluent;
- A second part with a test strip (14);
- The first and second part being provided with cooperating first and second coupling elements;
- A filter (12) for filtering blood cells from a blood sample (B) diluted with the diluent, which may be provided in the first (2) or second part (3), or as separate element for coupling with one of these parts (2, 3), through which at least blood plasma can pass but through which blood cells cannot pass;
- An adsorbent and/or absorbent element (17) which provides for the possibility of sampling a known quantity of blood, which adsorbent and/or absorbent element (17) is provided in the first part (2, 3), or as separate element for coupling with one of these parts (2, 3), whereby the blood constituents can be diluted by the diluent passed through the adsorbent and/or absorbent element (17).

19. Kit of parts according to claim 18, wherein the absorbing and/or adsorbing element (17) is provided with a reactive component, especially a component reactive with components of the sample (B) and/or components on, in or of the test strip (14).

20. Kit of parts according to claim 19, wherein at least two absorbing and/or adsorbing elements (17) are provided, with different reactive components.

## Patentansprüche

1. Testvorrichtung (1) zum Testen einer menschlichen oder tierischen Probe, wie etwa Blut oder Blutbestandteile (B), umfassend ein erstes Teil (2) und ein zweites Teil (3), umfassend zusammenarbeitende erste und zweite Kopplungselemente (4, 5) zum Koppeln des ersten Teils (2) mit dem zweiten Teil (3), wobei das erste Teil (2) ein Flüssigkeitsreservoir (20) umfasst und wobei das zweite Teil (3) mindestens ein Behältnis umfasst, **dadurch gekennzeichnet, dass** ein Probe absorbierendes und/oder adsorbierendes Element (17) bereitgestellt ist, das in dem ersten Teil befestigt ist oder in das erste Teil eingeführt werden kann, wobei das Flüssigkeitsreservoir (20) im Volumen reduzierbar ist, um eine darin enthaltene Flüssigkeit durch das und/oder an dem adsorbierenden und/oder absorbierenden Element (17) entlang zu zwingen, was die Probe wie etwa Blut oder Bestandteile (B) davon aus dem adsorbierenden und/oder absorbierenden Element (17) in das Behältnis zwingt, bevorzugt in Kontakt mit mindestens einem Reagens in dem zweiten Teil (3), wenn das erste und das zweite Teil (2, 3) verbunden sind oder von mindestens den ersten und zweiten Kopplungselementen (4, 5) miteinander in Verbindung gebracht werden.

2. Testvorrichtung nach Anspruch 1, wobei das erste Teil (2) mit dem ersten Kopplungselement (4) an oder nahe einem ersten Ende (7) davon versehen ist, wobei das adsorbierende und/oder absorbierende Element (17) an oder nahe dem ersten Ende (7) bereitgestellt ist, und wobei das Flüssigkeitsreservoir (20) an einer Seite des adsorbierenden und/oder absorbierenden Elements (17) gegenüber dem ersten Ende (7) bereitgestellt ist.

3. Testvorrichtung nach Anspruch 1 oder 2, wobei die ersten und zweiten Kopplungselemente (4, 5) für lösbare Positionierung des ersten Teils (2) zu dem zweiten Teil (3) konzipiert sind, sodass ein erstes Ende (7) des ersten Teils (2) über einem Einlass eines Kanals (13) in dem zweiten Teil (3) positioniert wird, wobei in dem Kanal (13) mindestens ein Filter (12) in dem Einlass oder zwischen dem Einlass und dem mindestens einen Reagens (16) bereitgestellt ist.

4. Testvorrichtung nach einem der vorhergehenden Ansprüche, wobei das Flüssigkeitsreservoir (20) eine Kammer (24) und einen Kolben (25) umfasst, beweglich zu der Kammer (24), um das Volumen des Reservoirs (20) zu reduzieren, wobei ein Auslass für das Reservoir (20) bereitgestellt ist, der sich zu dem mindestens einen adsorbierenden und/oder absorbierenden Element (17) öffnet.

5. Testvorrichtung nach Anspruch 4, wobei das erste Teil (2) mit einem Griff (26) versehen ist, drehbar an dem oder um das Gehäuseteil (27) des ersten Teils (2), umfassend mindestens die Kammer (24) und/oder den Kolben (25), sodass eine Drehung des Griffs (26) in einer Bewegung des Kolbens (25) zu der Kammer (24) zum Reduzieren des Volumens resultiert.

6. Testvorrichtung nach Anspruch 5, wobei der Griff (26) mit mindestens einem Zahn (28) versehen ist und das Gehäuseteil (27) mit einer Führungsspur (29) für den Zahn (28) oder umgekehrt, wobei die Führungsspur (29) so verläuft, dass eine Drehung des Griffs (26) zu einer erzwungenen Bewegung des Griffs (26) in einer linearen Richtung, bevorzugt einer Bewegung zu oder weg von dem adsorbierenden und/oder absorbierenden Element (17) führt.

7. Testvorrichtung nach einem der vorhergehenden Ansprüche, wobei das zweite Teil (3) mindestens einen Teststreifen (14) umfasst, bevorzugt mit einer Längsrichtung, wobei das mindestens eine Reagens (16) in oder auf dem Streifen (14) bereitgestellt wird und wobei der Streifen (14) von außerhalb des zweiten Teils (3) mindestens teilweise sichtbar ist, bevorzugt mindestens ein Teil des Streifens (14) umfassend das mindestens eine Reagens (16).

8. Testvorrichtung nach einem der vorhergehenden Ansprüche, wobei das erste Teil (2) eine erste Längsrichtung hat und das zweite Teil (3) eine zweite Längsrichtung hat, wobei die ersten und zweiten Längsrichtungen in einem Winkel zueinander verlaufen, der anders als 0 oder 180 Grad und bevorzugt im Wesentlichen 90 Grad ist.

9. Testvorrichtung nach Anspruch 8, wobei das erste Teil (2) ein erstes Kopplungselement (4) hat, umfassend oder gebildet von einem im Wesentlichen zylindrischen Teil, während das zweite Teil (3) ein zweites Kopplungselement (5) hat, umfassend eine mindestens teilweise kreisförmige Wand, verlaufend im Wesentlichen senkrecht zu der zweiten Längsrichtung, bildend eine Öffnung zum Bilden eines Schlosses, bevorzugt bildend mindestens ein formschlüssiges Schloss und/oder ein Presspassungsschloss mit dem ersten Kopplungselement (4), sodass das erst Kopplungselement (4) in das zweite Kopplungselement (5) in der ersten Längsrichtung eingesetzt werden kann, um das erste (2) mit dem zweiten Teil (3) zu koppeln.

10. Testvorrichtung nach einem der vorhergehenden Ansprüche, wobei das erste Teil (2) eine Dichtung (46) umfasst, die das Reservoir (20) abdichtet, wobei das erste Teil (2) mit einem Element (52) zum Durchstechen oder Brechen der Dichtung (46) versehen ist, sodass eine Flüssigkeitsverbindung zwischen dem Reservoir (20) und dem adsorbierenden und/oder absorbierenden Element (17) gebildet wird.

11. Testvorrichtung nach einem der vorhergehenden Ansprüche, wobei das erste und zweite Teil (2, 3) von den ersten und zweiten Kopplungselementen (4, 5) gekoppelt werden, wobei, mit dem Reservoir (20) in einer maximal reduzierten Volumenposition, eine Flüssigkeitsverbindung bereitgestellt wird zwischen dem Reservoir (20) und dem mindestens einen Reagens (16) durch das adsorbierende und/oder absorbierende Element (17) und einem Filter (12), bereitgestellt zwischen dem Element (17) und dem mindestens einen Reagens (16), wobei die Flüssigkeitsverbindung im Wesentlichen vollständig mit einer Flüssigkeit aus dem Reservoir (20) und darin enthaltenen Probenbestandteilen, bevorzugt mindestens Blutplasma, gefüllt ist, während der Filter (12) bevorzugt ein Filter ist, geeignet zum Filtern von mindestens Blutzellen aus dem Blut vor dem Kontakt mit dem mindestens einen Reagens (16).

12. Testvorrichtung nach einem der vorhergehenden Ansprüche, wobei der Filter (12) bereitgestellt wird, um mindestens Blutzellen aus Blut oder Teilchen aus einer Probe (B) zu filtern, welcher Filter (12) ein einzelner, doppelter oder mehrschichtiger Filter ist.

13. Testvorrichtung nach einem der vorhergehenden Ansprüche, wobei das erste Teil (2) einen Mechanismus zum Öffnen des Reservoirs (20) und kontrollierten Reduzieren des Volumens des Reservoirs (20) umfasst, um Flüssigkeit aus dem Reservoir (20) zu zwingen.

14. Testvorrichtung, bevorzugt nach einem der vorhergehenden Ansprüche, umfassend ein Blut absorbierendes und/oder adsorbierendes Element (17) zum Nehmen einer Blutprobe (B) und bevorzugt einen Filter (12) zum Herausfiltern von Elementen aus der Blutprobe (B), wobei mindestens eines von dem Blut absorbierenden und/oder adsorbierenden Element (17) und dem Filter (12) mit einer reaktiven Komponente versehen ist, reaktiv mit Bestandteilen der Probe (B) und/oder Bestandteilen auf, in oder von einem Teststreifen (14).

15. Verfahren zum Testen von menschlichen oder tierischen Proben, wie etwa Blut oder Blutbestandteilen davon, wobei eine Menge einer Probe (B) in und/oder auf einem Element (17) adsorbiert und/oder absorbiert wird, das an einer Seite mit einem Reservoir (20) verbunden ist, das eine Flüssigkeit enthält, besonders ein Lösungsmittel oder Verdünnungsmittel, wobei das Element (17) mit einem Kanal (13) verbunden ist, umfassend mindestens ein Reagens (16) und einen Filter (12), wobei, wenn ein erstes und ein zweites Teil, umfassend zusammenarbeitende erste und zweite Kopplungselemente zum Koppeln des ersten Teils mit dem zweiten Teil und das zweite Element den Kanal umfasst, von den mindestens ersten und zweiten Kopplungselementen miteinander verbunden oder in Kontakt gebracht werden, das Reservoir (20) im Volumen so reduziert wird, dass die darin enthaltene Flüssigkeit mindestens teilweise durch das und/oder entlang dem Element (17) gezwungen wird, sodass die darin und/oder darauf bereitgestellte Probe aufgelöst und/oder verdünnt wird, und die dann verdünnte und/oder aufgelöste Probe (B) mindestens teilweise durch den Filter (12), wobei in dem Filter (12) mindestens Zellen herausgefiltert werden, bevorzugt mindestens Blutzellen, während der Teil der verdünnten und/oder aufgelösten Probe (B), passierend durch den Filter (12), mindestens teilweise in Kontakt mit dem mindestens einen Reagens (16) gebracht wird.

16. Verfahren nach Anspruch 15, wobei das Element (17) zum Adsorbieren und/oder Adsorbieren von Blut in einem ersten Teil (2) einer Testvorrichtung (1) neben dem Reservoir (20) enthalten ist, welches Element (17) im Wesentlichen mit Blut gesättigt ist, wonach das erste Teil (2) mit einem zweiten Teil (3) der Vorrichtung (1), umfassend das mindestens eine Reagens (16), verbunden wird, wonach das Reservoir (20) im Volumen reduziert wird, was die Flüssigkeit aus dem Reservoir (20) durch das Element (17) und in und durch das zweite Teil (3) in Kontakt mit dem mindestens einen Reagens (16) zwingt.

17. Verfahren zur Herstellung einer menschlichen oder tierischen Probe zum Testen, wobei eine Menge der Probe (B) in und/oder auf einem Element (17), das an einer Seite verbunden ist mit einem Reservoir (20), enthaltend eine Flüssigkeit, insbesondere ein Lösungsmittel oder Verdünnungsmittel, adsorbiert und/oder absorbiert wird, wobei das Element (17) mit einem Kanal (13) verbunden ist, umfassend mindestens ein Reagens (16) und einen Filter (12), wobei, wenn ein erstes und ein zweites Teil, umfassend zusammenarbeitende erste und zweite Kopplungselemente zum Koppeln des ersten Teils mit dem zweiten Teil und das zweite Teil den Kanal umfasst, von mindestens dem ersten und zweiten Kopplungselement miteinander verbunden oder in Verbindung miteinander gebracht werden, das Reservoir (20) im Volumen reduziert wird, sodass die darin enthaltene Flüssigkeit mindestens teilweise durch das und/oder an dem Element (17) entlang gezwungen wird, was die darin und/oder darauf bereitgestellte Probe (B) aufgelöst und/oder verdünnt, und die dann verdünnte und/oder aufgelöste Probe (B) mindestens teilweise durch den Filter (12), wobei in dem Filter (12) mindestens Zellen, bevorzugt mindestens Blutzellen, heraus gefiltert werden, während der Teil der verdünnten und/oder aufgelösten Probe (B), passierend durch den Filter (12), in einem Behältnis zur Lagerung und/oder zum Transport aufgenommen wird.

18. Kit von Teilen für eine Testvorrichtung nach einem der Ansprüche 1-14 oder zum Ausführen eines Verfahrens nach einem der Ansprüche 15-17, umfassend:
- ein erstes Teil (2) mit einem Verdünnungsmittel;
- ein zweites Teil mit einem Teststreifen (14);
- das erste und zweite Teil versehen mit zusammenarbeitenden ersten und zweiten Kopplungselementen;
- einen Filter (12) zum Filtern von Blutzellen aus einer mit dem Verdünnungsmittel verdünnten Blutprobe (B), der in dem ersten (2) oder zweiten Teil (3) oder als separates Element zum Koppeln mit einem dieser Teile (2, 3) bereitgestellt sein kann, durch den mindestens Blutplasma passieren kann aber durch den Blutzellen nicht passieren können;
- ein adsorbierendes und/oder absorbierendes Element (17), das für die Möglichkeit der Probenahme einer bekannten Menge Blut sorgt, welches adsorbierende und/oder adsorbierende Element (17) in dem ersten Teil (2, 3) oder als separates Element zum Koppeln mit einem dieser Teile (2, 3) bereitgestellt ist, wodurch die Blutbestandteile von dem Verdünnungsmittel, passiert durch das adsorbierende und/oder absorbierende Element (17), verdünnt werden können.

19. Kit von Teilen nach Anspruch 18, wobei das absorbierende und/oder adsorbierende Element (17) versehen ist mit einer reaktiven Komponente, besonders einer Komponente, reaktiv mit Bestandteilen der Probe (B) und/oder Bestandteilen auf, in oder von dem Teststreifen (14).

20. Kit von Teilen nach Anspruch 19, wobei mindestens zwei absorbierende und/oder adsorbierende Elemente (17) bereitgestellt sind, mit verschiedenen reaktiven Komponenten.

## Revendications

1. Dispositif de test (1) pour tester un échantillon humain ou animal, tel que du sang ou des composants sanguins (B), comprenant une première partie (2) et une seconde partie (3), comprenant des premier et second éléments de couplage (4, 5) coopératifs pour coupler la première partie (2) avec la seconde partie (3), dans lequel la première partie (2) comprend un réservoir de fluide (20), et dans lequel la seconde partie (3) comprend au moins un réceptacle, **caractérisé en ce que** l'on prévoit un élément d'absorption et/ou d'adsorption d'échantillon (17) qui est fixé dans la première partie ou peut être introduit dans la première partie, dans lequel le réservoir de fluide (20) est réductible en volume pour forcer un liquide contenu à l'intérieur à travers et/ou le long de l'élément d'adsorption et/ou d'absorption (17), forçant l'échantillon tel que du sang ou ses composants (B) de l'élément d'adsorption et/ou d'absorption (17) dans le réceptacle, de préférence en contact avec au moins un réactif dans la seconde partie (3) lorsque les première et seconde parties (2, 3) sont raccordées ou amenées en raccordement entre elles par au moins les premier et second éléments de couplage (4, 5).

2. Dispositif de test selon la revendication 1, dans lequel la première partie (2) est prévue avec les premiers éléments de couplage (4) au niveau de ou à proximité de sa première extrémité (7), dans lequel l'élément d'adsorption et/ou d'absorption (17) est prévu au niveau de ou à proximité de ladite première extrémité (7), et dans lequel le réservoir de fluide (20) est prévu au niveau d'un côté de l'élément d'adsorption et/ou d'absorption (17) opposé à la première extrémité (7).

3. Dispositif de test selon la revendication 1 ou 2, dans lequel les premier et second éléments de couplage (4, 5) sont conçus pour le positionnement amovible de la première partie (2) par rapport à la seconde partie (3), de sorte qu'une première extrémité (7) de la première partie (2) est positionnée sur une entrée d'un canal (13) dans la seconde partie (3), dans lequel, dans le canal (13), au moins un filtre (12) est prévu dans l'entrée ou entre l'entrée et le au moins un réactif (16).

4. Dispositif de test selon l'une quelconque des revendications précédentes, dans lequel le réservoir de fluide (20) comprend une chambre (24) et un piston plongeur (25), mobile par rapport à ladite chambre (24) pour réduire le volume du réservoir (20), dans lequel une sortie est prévue pour le réservoir (20), s'ouvrant vers le au moins un élément d'adsorption et/ou d'absorption (17).

5. Dispositif de test selon la revendication 4, dans lequel la première partie (2) est prévue avec une poignée (26) pouvant tourner sur ou autour d'une partie de logement (27) de la première partie (2) comprenant au moins la chambre (24) et/ou le piston plongeur (25), de sorte qu'une rotation de ladite poignée (26) se traduit par un mouvement du piston plongeur (25) par rapport à la chambre (24), pour réduire ledit volume.

6. Dispositif de test selon la revendication 5, dans lequel la poignée (26) est prévue avec au moins une dent (28) et la partie de logement (27) avec un rail de guidage (29) pour ladite dent (28) et vice versa, dans lequel le rail de guidage (29) s'étend de sorte qu'une rotation de la poignée (26) mène à un mouvement forcé de la poignée (26) dans une direction linéaire, de préférence un mouvement vers ou à distance de l'élément d'adsorption et/ou d'absorption (17).

7. Dispositif de test selon l'une quelconque des revendications précédentes, dans lequel la seconde partie (3) comprend une bande de test (14), ayant de préférence une direction longitudinale, dans lequel le au moins un réactif (16) est prévu dans ou sur la bande (14), et dans lequel la bande (14) est au moins partiellement visible de l'extérieur de la seconde partie (3), de préférence au moins une partie de la bande (14) comprenant le au moins un réactif (16).

8. Dispositif de test selon l'une quelconque des revendications précédentes, dans lequel la première partie (2) a une première direction longitudinale et la seconde partie (3) a une seconde direction longitudinale, dans lequel les première et seconde directions longitudinales s'étendent à un angle mutuel différent de 0 ou de 180 degrés et de préférence sensiblement de 90 degrés.

9. Dispositif de test selon la revendication 8, dans lequel la première partie (2) a un premier élément de couplage (4), comprenant ou étant formé par une partie sensiblement cylindrique, alors que la seconde partie (3) a un second élément de couplage (5) comprenant une paroi au moins partiellement circulaire, s'étendant sensiblement perpendiculairement à la seconde direction longitudinale, formant une ouverture pour former un verrouillage, de préférence au moins un verrouillage de forme et/ou un verrouillage à ajustement serré, avec le premier élément de couplage (4), de sorte que le premier élément de couplage (4) peut être inséré dans le second élément de couplage (5) dans la première direction longitudinale pour coupler la première (2) avec la seconde partie (3).

10. Dispositif de test selon l'une quelconque des revendications précédentes, dans lequel la première partie (2) comprend un joint d'étanchéité (46) scellant le réservoir (20), dans lequel la première partie (2) est prévue avec un élément (52) pour percer ou rompre le joint d'étanchéité (46), de sorte qu'un raccordement de fluide est formé entre le réservoir (20) et l'élément d'adsorption et/ou d'absorption (17).

11. Dispositif de test selon l'une quelconque des revendications précédentes, dans lequel les première et seconde parties (2, 3) sont couplées par les premier et second éléments de couplage (4, 5), dans lequel avec le réservoir (20) dans une position de volume réduit au maximum, un raccordement de fluide est prévu entre le réservoir (20) et le au moins un réactif (16) à travers l'élément d'adsorption et/ou d'absorption (17) et un filtre (12) prévu entre l'élément (17) et le au moins un réactif (16), dans lequel le raccordement de fluide est sensiblement entièrement rempli avec un fluide provenant du réservoir (20) et des composants d'échantillon contenus à l'intérieur de ce dernier, de préférence au moins du plasma sanguin, alors que le filtre (12) est de préférence un filtre approprié pour filtrer au moins des cellules sanguines du sang avant d'entrer en contact avec le au moins un réactif (16).

12. Dispositif de test selon l'une quelconque des revendications précédentes, dans lequel un filtre (12) est prévu pour filtrer au moins des cellules sanguines du sang ou des particules d'un échantillon (B), lequel filtre (12) est un filtre simple, double ou multicouche.

13. Dispositif de test selon l'une quelconque des revendications précédentes, dans lequel la première partie (2) comprend un mécanisme pour ouvrir le réservoir (20) et la réduction contrôlée du volume du réservoir (20) pour forcer le liquide à partir du réservoir (20).

14. Dispositif de test selon l'une quelconque des revendications précédentes, comprenant un élément d'adsorption et/ou d'absorption de sang (17), pour prélever un échantillon de sang (B) et de préférence un filtre (12) pour filtrer les éléments de l'échantillon (B), dans lequel au moins l'un parmi l'élément d'adsorption et/ou d'absorption de sang (17) et le filtre (12) est prévu avec un composant réactif réagissant avec les composants de l'échantillon (B) et/ou les composants sur, dans ou d'une bande de test (14).

15. Procédé pour tester des échantillons humains ou animaux, tels que du sang ou ses composants, dans lequel une quantité d'un échantillon (B) est adsorbée et/ou absorbée dans et/ou sur un élément (17) qui, d'un côté, est raccordé à un réservoir (20) contenant un liquide, en particulier un solvant ou un diluant, dans lequel l'élément (17) est raccordé à un canal (13) comprenant au moins un réactif (16) et un filtre (12), dans lequel lorsqu'une première et une seconde partie, comprenant des premier et second éléments de couplage coopératifs pour coupler la première partie avec la seconde partie et la seconde partie comprenant le canal, sont raccordées ou amenées en raccordement entre elles par au moins les premier et second éléments de couplage, le réservoir (20) est réduit en volume, de sorte que le liquide contenu à l'intérieur de ce dernier est au moins partiellement forcé à travers et/ou le long de l'élément (17), dissolvant et/ou diluant l'échantillon (B) prévu à l'intérieur et/ou sur ce dernier, et l'échantillon est dilué et/ou dissout (B) ensuite au moins partiellement à travers le filtre (12), dans lequel dans le filtre (12), au moins des cellules sont filtrées, de préférence au moins des cellules sanguines, alors que la partie de l'échantillon dilué et/ou dissout (B) passant par le filtre (12) est au moins partiellement amenée en contact avec le au moins un réactif (16).

16. Procédé selon la revendication 15, dans lequel l'élément (17) pour adsorber et/ou absorber le sang est contenu dans une première partie (2) d'un dispositif de test (1), à côté du réservoir (20), lequel élément (17) est sensiblement saturé avec le sang, après quoi la première partie (2) est raccordée à une seconde partie (3) du dispositif (1), comprenant le au moins un réactif (16), après quoi le réservoir (20) est réduit en volume, forçant le liquide à partir du réservoir (20) à travers l'élément (17) et dans et à travers la seconde partie (3) en contact avec le au moins un réactif (16).

17. Procédé pour préparer un échantillon humain ou animal destiné à être testé, dans lequel une quantité d'un échantillon (B) est adsorbée et/ou absorbée dans et/ou sur un élément (17) qui, d'un côté, est raccordé à un réservoir (20) contenant un liquide, en particulier un solvant ou un diluant, dans lequel l'élément (17) est raccordé à un canal (13) comprenant au moins un réactif (16) et un filtre (12), dans lequel lorsqu'une première et une seconde partie, comprenant des premier et second éléments de couplage coopératifs pour coupler la première partie avec la seconde partie et la seconde partie comprenant le canal, sont raccordées ou amenées en raccordement entre elles par au moins les premier et second éléments de couplage, le réservoir (20) est réduit en volume, de sorte que le liquide contenu à l'intérieur est au moins partiellement forcé à travers et/ou le long de l'élément (17), dissolvant et/ou diluant l'échantillon (B) prévu à l'intérieur de ce dernier et/ou sur ce dernier, et l'échantillon est dilué et/ou dissout (B) ensuite au moins partiellement à travers le filtre (12), dans lequel dans le filtre (12), au moins des cellules sont filtrées, de préférence au moins des cellules sanguines, alors que la partie de l'échantillon dilué et/ou dissout (B) passant par le filtre (12) est reçue dans un réceptacle pour le stockage et/ou le transport.

18. Kit de pièces pour un dispositif de test selon l'une quelconque des revendications 1 à 14 ou pour réaliser un procédé selon l'une quelconque des revendications 15 à 17, comprenant :
une première partie (2) avec un diluant ;
une seconde partie avec une bande de test (14) ;
les première et seconde parties étant prévues avec des premier et second éléments de couplage coopératifs ;
un filtre (12) pour filtrer les cellules sanguines d'un échantillon de sang (B) dilué avec le diluant, qui peut être prévu dans la première (2) ou la seconde partie (3), ou sous forme d'élément séparé pour se coupler avec l'une de ces parties (2, 3), à travers lequel au moins du plasma sanguin peut passer, mais à travers lequel les cellules sanguines ne peuvent pas passer ;
un élément d'adsorption et/ou d'absorption (17) qui fournit la possibilité d'échantillonner une quantité connue de sang, lequel élément d'adsorption et/ou d'absorption (17) est prévu dans la première partie (2, 3), ou sous forme d'élément séparé pour se coupler avec l'une de ces parties (2, 3), moyennant quoi les constituants sanguins peuvent être dilués par le diluant qui passe à travers l'élément d'adsorption et/ou d'absorption (17).

19. Kit de pièces selon la revendication 18, dans lequel l'élément d'adsorption et/ou d'absorption (17) est prévu avec un composant réactif, en particulier un composant réagissant avec les composants de l'échantillon (B) et/ou les composants sur, dans ou de la bande de test (14).

20. Kit de pièces selon la revendication 19, dans lequel on prévoit au moins deux éléments d'adsorption et/ou d'absorption (17) avec des composants réactifs différents.
